(19) Europäisches Patentamt
European Patent Office
Office européen des brevets

(11) Publication number: **0 476 933 A1**

(12) **EUROPEAN PATENT APPLICATION**

(21) Application number: **91308360.6**

(22) Date of filing: **12.09.91**

(51) Int. Cl.$^5$: **C07K 7/06**, A61K 37/02

(30) Priority: **12.09.90 JP 243003/90**

(43) Date of publication of application:
**25.03.92 Bulletin 92/13**

(84) Designated Contracting States:
**AT BE CH DE DK ES FR GB GR IT LI LU NL SE**

(71) Applicant: **SUMITOMO PHARMACEUTICALS COMPANY, LIMITED**
**2-8, Dosho-Machi 2-Chome, Chuo-Ku**
**Osaka-Shi Osaka-Fu (JP)**

(71) Applicant: **Ojika, Kosei**
**18, Aza Miyashinki, Ooaza Kiori, Miwa-cho**
**Ama-gun, Aichi-ken (JP)**

(71) Applicant: **Yamamoto, Masahiko**
**21 Shirakabe-4-chome, Higashi-ku**
**Nagoya-shi (JP)**

(72) Inventor: **Ueki, Yasuyuki**
**4-2-302, Ryodocho**
**Nishinomiya-shi (JP)**
Inventor: **Fukushima, Nobuyuki**
**11-7-406, Sonehigashinocho-2-chome**
**Toyonaka-shi (JP)**
Inventor: **Ikeda, Yoshiharu**
**5 Prichard Avenue**
**Somerville, MA 02144 (JP)**
Inventor: **Nishihara, Toshio**
**20-7, Kamihamuro-5-chome**
**Takatsuki-shi (JP)**
Inventor: **Ono, Keiichi**
**10-4, Momoyamadai-3-cho**
**Sakai-shi (JP)**
Inventor: **Irie, Tsunemasa**
**6-12, Mukoyama-2-chome**
**Takarazuka-shi (JP)**

(74) Representative: **Cresswell, Thomas Anthony et al**
**J.A. Kemp & Co. 14 South Square Gray's Inn**
**London WC1R 5LX (GB)**

# (54) Neurotrophic peptide derivatives.

(57) Neurotrophic peptides such as

H-Ala-Ala-Asp-Ile-Ser-Gln-D-Trp-Ala-Gly-Pro-Leu-OH

H-Ile-Ser-Gln-D-Trp-Ala-$NH_2$

H-MeIle-Ser-Gln-D-Trp-Ala-$NH(CH_2)_3NH_2$

H-MeIle-Ser-Gln-D-Trp-Ala-$NH(CH_2)_3N(CH_3)_2$

H-MeIle-Ser-Gln-D-Trp-Ala-$NH(CH_2)_4NHC(=NH)NH_2$

$Me_2$Ile-Ser-Gln-D-Trp-Ala-$NH(CH_2)_8NH_2$

$Me_3$Ile-Ser-Gln-D-Trp-Ala-$NH(CH_2)_8NH_2$

pGlu-Trp-OH

H-Ala-Cys-Asp-Ile-Ser-Gln-Trp-Cys-$NH_2$

H-Ala-Ala-Asp-Cys-Ser-Gln-Trp-Cys-$NH_2$

Ac-Ala-Asp-Ile-Ser-Gln-Trp-Lys-$NH_2$

Ac-Ala-Glu-Ile-Ser-Gln-Trp-Lys-$NH_2$

EP 0 476 933 A1

are useful in treating neuro-degenerate disorders and dementia.

## BACKGROUND OF THE INVENTION

### Field of the Invention

The present invention relates to neurotrophic peptide derivatives which are useful as drugs. The invention also relates to compositions comprising the derivatives as effective components for the treatment of neurodegenerative diseases.

### Related Art Statement

Neurotrophic factors are generally supplied from target organ in neuro-terminal, glia cell or blood. The factors are known to have biological activities for survival and maintenance of neuronal cell, acceleration of neurite elongation, and for induction of an enzyme for synthesizing neuro-transmitters. Especially, in the formation and maintenance of neuronal network with long distance projection, the supplement of neurotrophic factor from target cell is considered to be indispensable. The relation between neuronal degeneration or denervation and neurological disease has been made clearer. As typical example, it is suggested that the degeneration/denervation of cholinergic neuron from basal forebrain to hippocampal neocortex is associated with Alzheimer type dementia. There are also suggested the relation between the degeneration/denervation of dopaminergic neuron from substantia nigra to striatum and Parkinson's disease, and the relation between the degeneration/denervation of spinal motor-neuron and amyotrophic lateral sclerosis. These degeneration/denervation, i.e., the cause of these diseases is supposed to be due to the decrease or deficiency in the supplement of neurotrophic factor from target cell, so that there is suggested a possibility for the treatment of neurological degenerative disorders by the supplement of neurotrophic factors [Appel. S.H., Annals of Neurology, 10, 499 (1981)].

Many reports have shown evidence of the existence of neurotrophic factors in mammals. However, nerve growth factor (NGF), brain-derived neurotrophic factor (BDNF) and ciliary neurotrophic factor (CNTF) have been hitherto isolated as neurotrophic factors and analysed for the primary structures.

NGF is composed of three different subunits α, β and γ with the constitutional ratio of $\alpha_2\beta\gamma_2$. It is reported that only βNGF exhibits the biological activity [Varon, S. et al., Biochemistry, 7, 1296 (1968)]. The primary structure of mouse β-NGF was determined by Angeletti & Bradshow in 1971 to reveal that β-NGF is a protein composed of 118 amino acids [molecular weight (Mw); 13,259)], [Angeletti & Bradshow, Proc. Natl. Acad. Sci. USA, 68, 2417 (1971)]. NGF is involved in sympathetic neuronal and sensory neuronal systems at peripheral neuronal system, and also recognized to be involved in cholinergic neuronal system at the central neuronal systems [Korsching, S. et al., Neuroscience Letters, 54, 201, (1985); Korsching, S. et al., EMBO Journal, 4, 1389, (1985)].

BDNF was purified by Thoenen et al. from hog brain as a neurotrophic factor for survival of sensory neurons in embryo dorsal root ganglion, and the primary structure of BDNF was recently reported by Barde et al. to show that hog BDNF is a protein having 119 amino acids in total (Mw; 13,511) and having very high homology with NGF [Barde, Y.A. et al., EMBO J., 1, 549 (1982); Leiblock J. et al., Nature, 341, 149 (1989)].

CNTF is a neurotrophic factor which was purified from chick embryo eyes by Manthorpe et al. and has an activity for survival of parasympathetic ganglion (cilliary ganglion) cells. Recently, the primary structure of CNTF has been determined by Collins et al., using CNTF purified from rabbit sciatic nerve. According to the report, rabbit CNTF is a protein having the total amino acid number of 199 (Mw: 22,660) [Manthorpe, M. et al., Journal of Neurochemistry, 34, 69 (1980); Manthorpe, M. et al., Journal of Neuroscience Research, 8, 233 (1982); Manthorpe, M. et al., Federation Proceedings, 44, 2753 (1985)] [Collins F. et al., Science, 246, 1023 (1989)].

Furthermore, paying attention to high similarity between NGF and BDNF, survey of a third similar factor has been made by 3 research groups and succeeded in cloning of a factor called neurotrophin-3 (NT-3) or nerve growth factor-2 (NGF-2). According to the survey, the third factor is a protein having the total amino acid number of 119, the structure of which is preserved in mouse, rat and human [Hohn A. et al., Nature, 344, 339 (1990)] [Maisonpierre P.C. et al., Science, 247, 1446 (1990)] [Kaisho Y. et al., FEBS Letters, 266, 187 (1990)].

In addition to the neurotrophic factors described above, other several neurotrophic factors have been reported, although they have not yet been highly purified. Among them, as neurotrophic factors which are derived from hippocampus or act on hippocampus or medial septum nuclei, some neurotrophic factors have been reported as follows:

(1) Neurotrophic factor partially purified from hippocampal tissue of neonatal rat (2-3 weeks old) [Ojika, K. et al., Proc. Natl. Acad. Sci. USA, 81, 2567 (1984); Appel. S.H., Japanese Patent KOKAI (Laid-Open) No. 58-154514; Ojika, K., Japanese Journal of Psycopharmacology, 7, 447 (1987)]:

The water soluble factor exists fairly and specifically in hippocampal tissue, and enhances the growth of

cultured medial septum nuclei cholinergic neuron. The results of partial purification reveal that this factor is a polypeptide having a molecular weight of about 900 daltons. The factor is considered to be different from NGF, because its activity is not inhibited by the addition of anti-NGF antibody [Bostwick, J.R. et al., Brain Research, 422, 92 (1987)].

(2) Neurotrophic factor derived from the conditioned medium of neonatal rat brain astrocyte [Muller, H.W. et al., Proc. Natl. Acad. Sci. USA, 81, 1248 (1984)]:

This factor is considered from elution position of gel filtration to have a molecular weight of about 500 and to be non-protein factor because of the fact that its activity is not lost even after the treatment with trypsin or pronase.

(3) Neurotrophic factor partially purified from hippocampal extract of rats whose fimbria fornics have been lesioned before 14 days [Yoshida, K., Keiou Igaku, 65, (1), 45, (1988)]:

Three factors having molecular weights of 10,000 to 20,000, 30,000 to 40,000 and 50,000 to 60,000, respectively, as measured by gel filtration have been reported.

(4) Neurotrophic factor derived from rat hippocampal tissue [Heacock, A.M. et al., Brain Research, 363, 299, (1986)]:

This factor exhibits an activity for survival of chick ciliary ganglion cells, and is an acidic protein having a molecular weight of 10,000 or more.

As mentioned above, these hippocampus-derived factors or factors which act on hippocampus or medial septum nuclei have been reported as proteinacious or non-proteinacious factors; however, none of them has been isolated and purified or further determined for their primary structures.

Moreover, NGF, BDNF and CNTF, already isolated and determined for the primary structures, are proteinacious factors having high molecular weights of 13,259, 13,511 and 22,660, respectively; cloned NT-3 (NGF-2) is also a protein having the total amino acid number of 119. In the case where such factors are used as agents for the treatment of neuro-degenerative disorder, the factors are likely to cause difficult problems about bioavailability, industrial production, etc. Therefore, it is desired to develop a peptidic neurotrophic factor having a lower molecular weight and its derivatives having a similar pharmacological activity.

## SUMMARY OF THE INVENTION

The present invention solves the foregoing problems in the prior art. That is, an object of the present invention is to provide novel neurotrophic peptide derivatives which have an activity for enhancing acetylcholine synthesis in cholinergic neurons.

Another object of the present invention is to provide compositions for the treatment of neuro-degenerative disorders.

In order to solve the foregoing problems, various investigations were made. Prior to the present invention, Ojika et al. already succeeded in isolating and purifying a novel neurotrophic peptide and determining its structure (Japanese Patent KOKAI (Laid-Open) No. 3-72499). The neurotrophic peptide is a peptidic neurotrophic factor which has an activity for enhancing acetylcholine synthesis in cholinergic neurons and has the following amino acid sequence:

$CH_3CO$- (or H-) Ala-Ala-Asp-Ile-Ser-Gln-Trp-Ala-Gly-Pro-Leu-OH

[hereafter the neurotrophic peptide is sometimes abbreviated as HCNP (Hippocampal Cholinergic Neurotrophic Peptide)].

As a result of further investigations, Ojika et al. found that a partial peptide of HCNP and derivatives obtained by modifying HCNP or its partial peptide at the N terminus and/or C terminus exhibit the HCNP activity almost similar to that of HCNP (Japanese Patent Application No. 1-280590).

The present inventors have further made investigations and as a result, unexpectedly found that derivatives obtained by substituting selected amino acid residues of HCNP or its selected partial peptide with other selected amino acid residues or amino acid derivatives, or by forming a cyclic structure via disulfide bond or amide bond on the side chain and optionally modifying the N terminus and/or the C terminus also exhibit the HCNP activity. The present invention has thus been accomplished.

Accordingly, one aspect of the present invention is directed to novel neurotrophic peptide derivatives represented by the formula (I):

$$A^6 - A^7 \quad (I)$$

[wherein:

(A) in the case where $A^6$ represents $A^5$-Gln and $A^7$ represents Trp-$A^8$;

(a) in the case where:

$A^5$ represents $A^4$-Ser,

$A^4$ represents $A^3$-Ile,

$A^3$ represents $A^2$-Asp and,
$A^2$ represents $A^1$-Ala;
$A^1$ represents X-D-Ala, X-Tyr, X-Arg or X-MeAla,
$A^8$ represents Y or Ala-$A^9$,
$A^9$ represents Y or Gly-$A^{10}$,
$A^{10}$ represents Y or Pro-$A^{11}$ and
$A^{11}$ represents Y or Leu-Y;
(b) in the case where $A^3$ to $A^5$ have the same significances as in (a) and $A^2$ represents $A^1$-Met, $A^1$-Met(O) or $A^1$-Met($O_2$);
$A^1$ represents X, X-Ala, X-D-Ala, X-Tyr, X-Arg or X-MeAla,
$A^8$ represents Y or Ala-$A^9$,
$A^9$ represents Y or Gly-$A^{10}$,
$A^{10}$ represents Y or Pro-$A^{11}$ and
$A^{11}$ represents Y or Leu-Y;
(c) in the case where $A^3$ to $A^5$ have the same significances as in (a) and $A^2$ represents $A^1$-Cys (provided that $A^1$-Cys forms a cyclic structure together with Cys-$A^9$ of $A^8$ via disulfide bond);
$A^1$ represents X, X-Ala, X-D-Ala, X-Tyr, X-Arg or X-MeAla,
$A^8$ represents Cys-$A^9$ (provided that Cys-$A^9$ forms a cyclic structure together with $A^1$-Cys of $A^2$ via disulfide bond),
$A^9$ represents Y or Gly-$A^{10}$,
$A^{10}$ represents Y or Pro-$A^{11}$ and
$A^{11}$ represents Y or Leu-Y;
(d) in the case where $A^4$ and $A^5$ have the same significances as in (a) and $A^3$ represents $A^2$-Asp (provided that $A^2$-Asp forms a cyclic structure together with Lys-$A^9$ of $A^8$ via amide bond);
$A^2$ represents X, $A^1$-Ala, $A^1$-Met, $A^1$-Met(O) or $A^1$-Met($O_2$),
$A^1$ represents X, X-Ala, X-D-Ala, X-Tyr, X-Arg or X-MeAla,
$A^8$ represents Lys-$A^9$ (provided that Lys-$A^9$ forms a cyclic structure together with $A^2$-Asp of $A^3$ via amide bond),
$A^9$ represents Y or Gly-$A^{10}$,
$A^{10}$ represents Y or Pro-$A^{11}$ and
$A^{11}$ represents Y or Leu-Y;
(e) in the case where $A^4$ and $A^5$ have the same significances as in (a), $A^3$ represents $A^2$-Gln and $A^2$ represents X, $A^1$-Ala, $A^1$-Met, $A^1$-Met(O) or $A^1$-Met($O_2$);
$A^1$ represents X, X-Ala, X-D-Ala, X-Tyr, X-Arg or X-MeAla,
$A^8$ represents Y or Ala-$A^9$,
$A^9$ represents Y or Gly-$A^{10}$,
$A^{10}$ represents Y or Pro-$A^{11}$ and
$A^{11}$ represents Y or Leu-Y;
(f) in the case where $A^3$ to $A^5$ have the same significances as in (e) and $A^2$ represents $A^1$-Cys (provided that $A^1$-Cys forms a cyclic structure together with Cys-$A^9$ of $A^8$ via disulfide bond);
$A^1$ represents X, X-Ala, X-D-Ala, X-Tyr, X-Arg or X-MeAla,
$A^8$ represents Cys-$A^9$ (provided that Cys-$A^9$ forms a cyclic structure together with $A^1$-Cys of $A^2$ via disulfide bond),
$A^9$ represents Y or Gly-$A^{10}$,
$A^{10}$ represents Y or Pro-$A^{11}$ and
$A^{11}$ represents Y or Leu-Y;
(g) in the case where $A^4$ and $A^5$ have the same significances as in (a), $A^3$ represents $A^2$-Glu and $A^2$ represents X, $A^1$-Ala, $A^1$-Met, $A^1$-Met(O) or $A^1$-Met($O_2$);
$A^1$ represents X, X-Ala, X-D-Ala, X-Tyr, X-Arg or X-MeAla,
$A^8$ represents Y or Ala-$A^9$,
$A^9$ represents Y or Gly-$A^{10}$,
$A^{10}$ represents Y or Pro-$A^{11}$ and
$A^{11}$ represents Y or Leu-Y;
(h) in the case where $A^3$ to $A^5$ have the same significances as in (g) and $A^2$ represents $A^1$-Cys (provided that $A^1$-Cys forms a cyclic structure together with Cys-$A^9$ of $A^8$ via disulfide bond);
$A^1$ represents X, X-Ala, X-D-Ala, X-Tyr, X-Arg or X-MeAla,
$A^8$ represents Cys-$A^9$ (provided that Cys-$A^9$ forms a cyclic structure together with $A^1$-Cys of $A^2$ via disulfide bond),

$A^9$ represents Y or Gly-$A^{10}$,
$A^{10}$ represents Y or Pro-$A^{11}$ and
$A^{11}$ represents Y or Leu-Y;
(i) in the case where $A^4$ and $A^5$ have the same significances as in (a) and $A^3$ represents $A^2$-Glu (provided that $A^2$-Glu forms a cyclic structure together with Lys-$A^9$ of $A^8$ via amide bond);
$A^2$ represents X, $A^1$-Ala, $A^1$-Met, $A^1$-Met(O) or $A^1$-Met($O_2$),
$A^1$ represents X, X-Ala, X-D-Ala, X-Tyr, X-Arg or X-MeAla,
$A^8$ represents Lys-$A^9$ (provided that Lys-$A^9$ forms a cyclic structure together with $A^2$-Glu of $A^3$ via amide bond),
$A^9$ represents Y or Gly-$A^{10}$,
$A^{10}$ represents Y or Pro-$A^{11}$ and
$A^{11}$ represents Y or Leu-Y;
(j) in the case where $A^5$ has the same significance as in (a),
$A^4$ represents $A^3$-Pro or $A^3$-Aib,
$A^3$ represents X or $A^2$-Gln and
$A^2$ represents X, $A^1$-Ala, $A^1$-Met, $A^1$-Met(O) or $A^1$-Met($O_2$);
$A^1$ represents X, X-Ala, X-D-Ala, X-Tyr, X-Arg or X-MeAla,
$A^8$ represents Y or Ala-$A^9$,
$A^9$ represents Y or Gly-$A^{10}$,
$A^{10}$ represents Y or Pro-$A^{11}$ and
$A^{11}$ represents Y or Leu-Y;
(k) in the case where $A^3$ to $A^5$ have the same significances as in (j) and $A^2$ represents $A^1$-Cys (provided that $A^1$-Cys forms a cyclic structure together with Cys-$A^9$ of $A^8$ via disulfide bond);
$A^1$ represents X, X-Ala, X-D-Ala, X-Tyr, X-Arg or X-MeAla,
$A^8$ represents Cys-$A^9$ (provided that Cys-$A^9$ forms a cyclic structure together with $A^1$-Cys of $A^2$ via disulfide bond),
$A^9$ represents Y or Gly-$A^{10}$,
$A^{10}$ represents Y or Pro-$A^{11}$ and
$A^{11}$ represents Y or Leu-Y;
(l) in the case where $A^4$ and $A^5$ have the same significances as in (j), $A^3$ represents $A^2$-Asp or $A^2$-Glu and
$A^2$ represents X, $A^1$-Ala, $A^1$-Met, $A^1$-Met(O) or $A^1$-Met($O_2$);
$A^1$ represents X, X-Ala, X-D-Ala, X-Tyr, X-Arg or X-MeAla,
$A^8$ represents Y or Ala-$A^9$,
$A^9$ represents Y or Gly-$A^{10}$,
$A^{10}$ represents Y or Pro-$A^{11}$ and
$A^{11}$ represents Y or Leu-Y;
(m) in the case where $A^3$ to $A^5$ have the same significances as in (1) and $A^2$ represents $A^1$-Cys (provided that $A^1$-Cys forms a cyclic structure together with Cys-$A^9$ of $A^8$ via disulfide bond);
$A^1$ represents X, X-Ala, X-D-Ala, X-Tyr, X-Arg or X-MeAla,
$A^8$ represents Cys-$A^9$ (provided that Cys-$A^9$ forms a cyclic structure together with $A^1$-Cys of $A^2$ via disulfide bond),
$A^9$ represents Y or Gly-$A^{10}$,
$A^{10}$ represents Y or Pro-$A^{11}$ and
$A^{11}$ represents Y or Leu-Y;
(n) in the case where $A^4$ and $A^5$ have the same significances as in (j) and $A^3$ represents $A^2$-Asp (provided that $A^2$-Asp forms a cyclic structure together with Lys-$A^9$ of $A^8$ via amide bond) or $A^2$-Glu (provided that $A^2$-Glu forms a cyclic structure together with Lys-$A^9$ of $A^8$ via amide bond);
$A^2$ represents X, $A^1$-Ala, $A^1$-Met, $A^1$-Met(O) or $A^1$-Met($O_2$),
$A^1$ represents X, X-Ala, X-D-Ala, X-Tyr, X-Arg or X-MeAla,
$A^8$ represents Lys-$A^9$ (provided that Lys-$A^9$ forms a cyclic structure together with $A^2$-Asp or $A^2$-Glu of $A^3$ via amide bond),
$A^9$ represents Y or Gly-$A^{10}$,
$A^{10}$ represents Y or Pro-$A^{11}$ and
$A^{11}$ represents Y or Leu-Y;
(o) in the case where $A^5$ has the same significance as in (a) and $A^4$ represents X-MeIle, $Me_2$Ile, $Me_3$Ile or AIle (N-amidinoisoleucine);
$A^8$ represents Y or Ala-$A^9$,

$A^9$ represents Y or Gly-$A^{10}$,
$A^{10}$ represents Y or Pro-$A^{11}$ and
$A^{11}$ represents Y or Leu-Y;
(p) in the case where $A^5$ has the same significance as in (a) and $A^4$ represents $A^3$-Cys (provided that $A^3$-Cys forms a cyclic structure together with Cys-$A^9$ of $A^8$ via disulfide bond);
$A^3$ represents X, $A^2$-Asp, $A^2$-Gln or $A^2$-Glu,
$A^2$ represents X, $A^1$-Ala, $A^1$-Met, $A^1$-Met(O) or $A^1$-Met($O_2$),
$A^1$ represents X, X-Ala, X-D-Ala, X-Tyr, X-Arg or X-MeAla,
$A^8$ represents Cys-$A^9$ (provided that Cys-$A^9$ forms a cyclic structure together with $A^3$-Cys of $A^4$ via disulfide bond),
$A^9$ represents Y or Gly-$A^{10}$,
$A^{10}$ represents Y or Pro-$A^{11}$ and
$A^{11}$ represents Y or Leu-Y;
(B) in the case where $A^6$ represents $A^5$-Gln and $A^7$ represents D-Trp-$A^8$;
(a) in the case where:
$A^5$ represents X or $A^4$-Ser,
$A^4$ represents $A^3$-Ile, $A^3$-Pro or $A^3$-Aib,
$A^3$ represents X or $A^2$-Gln and
$A^2$ represents X, $A^1$-Ala, $A^1$-Met, $A^1$-Met(O) or $A^1$-Met($O_2$);
$A^1$ represents X, X-Ala, X-D-Ala, X-Tyr, X-Arg or X-MeAla,
$A^8$ represents Y or Ala-$A^9$,
$A^9$ represents Y or Gly-$A^{10}$,
$A^{10}$ represents Y or Pro-$A^{11}$ and
$A^{11}$ represents Y or Leu-Y;
(b) in the case where $A^3$ to $A^5$ have the same significances as in (a) and $A^2$ represents $A^1$-Cys (provided that $A^1$-Cys forms a cyclic structure together with Cys-$A^9$ of $A^8$ via disulfide bond);
$A^1$ represents X, X-Ala, X-D-Ala, X-Tyr, X-Arg or X-MeAla,
$A^8$ represents Cys-$A^9$ (provided that Cys-$A^9$ forms a cyclic structure together with $A^1$-Cys of $A^2$ via disulfide bond),
$A^9$ represents Y or Gly-$A^{10}$,
$A^{10}$ represents Y or Pro-$A^{11}$ and
$A^{11}$ represents Y or Leu-Y;
(c) in the case where $A^4$ and $A^5$ have the same significances as in (a), $A^3$ represents $A^2$-Asp or $A^2$-Glu and
$A^2$ represents X, $A^1$-Ala, $A^1$-Met, $A^1$-Met(O) or $A^1$-Met($O_2$);
$A^1$ represents X, X-Ala, X-D-Ala, X-Tyr, X-Arg or X-MeAla,
$A^8$ represents Y or Ala-$A^9$,
$A^9$ represents Y or Gly-$A^{10}$,
$A^{10}$ represents Y or Pro-$A^{11}$ and
$A^{11}$ represents Y or Leu-Y;
(d) in the case where $A^3$ to $A^5$ have the same significances as in (c) and $A^2$ represents $A^1$-Cys (provided that $A^1$-Cys forms a cyclic structure together with Cys-$A^9$ of $A^8$ via disulfide bond);
$A^1$ represents X, X-Ala, X-D-Ala, X-Tyr, X-Arg or X-MeAla,
$A^8$ represents Cys-$A^9$ (provided that Cys-$A^9$ forms a cyclic structure together with $A^1$-Cys of $A^2$ via disulfide bond),
$A^9$ represents Y or Gly-$A^{10}$,
$A^{10}$ represents Y or Pro-$A^{11}$ and
$A^{11}$ represents Y or Leu-Y;
(e) in the case where $A^4$ and $A^5$ have the same significances as in (a) and $A^3$ represents $A^2$-Asp (provided that $A^2$-Asp forms a cyclic structure together with Lys-$A^9$ of $A^8$ via amide bond) or $A^2$-Glu (provided that $A^2$-Glu forms a cyclic structure together with Lys-$A^9$ of $A^8$ via amide bond);
$A^2$ represents X, $A^1$-Ala, $A^1$-Met, $A^1$-Met(O) or $A^1$-Met($O_2$),
$A^1$ represents X, X-Ala, X-D-Ala, X-Tyr, X-Arg or X-MeAla,
$A^8$ represents Lys-$A^9$ (provided that Lys-$A^9$ forms a cyclic structure together with $A^2$-Asp or $A^2$-Glu of $A^3$ via amide bond),
$A^9$ represents Y or Gly-$A^{10}$,
$A^{10}$ represents Y or Pro-$A^{11}$ and
$A^{11}$ represents Y or Leu-Y;

(f) in the case where $A^5$ has the same significance as in (a) and
$A^4$ represents X, X-MeIle, $Me_2$Ile, $Me_3$Ile or AIle (N-amidinoisoleucine);
$A^8$ represents Y or Ala-$A^9$,
$A^9$ represents Y or Gly-$A^{10}$,
$A^{10}$ represents Y or Pro-$A^{11}$ and
$A^{11}$ represents Y or Leu-Y;
(g) in the case where
$A^5$ has the same significance as in (a) and
$A^4$ represents $A^3$-Cys (provided that $A^3$-Cys forms a cyclic structure together with Cys-$A^9$ of $A^8$ via disulfide bond);
$A^3$ represents X, $A^2$-Asp, $A^2$-Gln or $A^2$-Glu,
$A^2$ represents X, $A^1$-Ala, $A^1$-Met, $A^1$-Met(O) or $A^1$-Met($O_2$),
$A^1$ represents X, X-Ala, X-D-Ala, X-Tyr, X-Arg or X-MeAla,
$A^8$ represents Cys-$A^9$ (provided that Cys-$A^9$ forms a cyclic structure together with $A^3$-Cys of $A^4$ via disulfide bond),
$A^9$ represents Y or Gly-$A^{10}$,
$A^{10}$ represents Y or Pro-$A^{11}$ and
$A^{11}$ represents Y or Leu-Y;
(C) in the case where $A^6$ represents pGlu and
$A^7$ represents Trp-$A^8$ or D-Trp-$A^8$;
$A^8$ represents Y or Ala-$A^9$,
$A^9$ represents Y or Gly-$A^{10}$,
$A^{10}$ represents Y or Pro-$A^{11}$ and
$A^{11}$ represents Y or Leu-Y; and in all cases of (A), (B) and (C);
X represents H, an acyl group, a sulfonyl group or a residue for forming a urea skeleton or urethane skeleton together with an amino group of the amino acid residue to which X is bound;
Y represents OH or a residue for forming an amide group or an ester group together with a carbonyl group of the amino acid residue to which Y is bound], or pharmaceutically acceptable salt thereof.

The present invention also provides pharmaceutical compositions for the treatment of neuro-degenerative disorders or dementia, comprising as an effective ingredient the neurotrophic peptide derivatives described above or salt thereof.

## DETAILED DESCRIPTION OF THE INVENTION

Hereafter the neurotrophic peptide derivatives of the present invention are described in more detail.

The neurotrophic peptide derivatives of the present invention refer to peptidic derivatives obtained by modifying or changing the structure of parent neurotrophic peptide (HCNP), i.e., by substitution of amino acid residues or cyclization via amino acid side chains and, if necessary, further followed by fragmentation and/or by N terminal and/or C-terminal modification.

The derivation by substitution of amino acid residues is characterized by selecting one or more sites from the 1, 2, 3, 4, 6 and 7-positions of HCNP for the substitution and then substituting these positions with amino acid residues or amino acid derivatives selected from the following groups:
the 1-position comprising D-Ala, Tyr, Arg and MeAla;
the 2-position comprising Met, Met(O) and Met($O_2$);
the 3-position comprising Gln and Glu,
the 4-position comprising Pro, Aib, MeIle, $Me_2$Ile, $Me_3$Ile and AIle (N-amidinoisoleucine),
the 6-position comprising pGlu, and,
the 7-position comprising D-Trp.

The cyclization via amino acid side chains is characterized by selecting the bond for forming the cyclic structure from an amide bond and a disulfide bond and substituting with Lys or Cys residue and, if necessary, after substitution with Glu residue, forming the cyclic structure via the selected bond. More specifically, where the cyclic structure is formed via the amide bond, Ala residue at the 8-position of HCNP is firstly replaced with Lys residue, if necessary, Asp residue at the 3-position is replaced with Glu residue and an amide bond is then formed between the side chain amino group of Lys residue at the 8-position and the side chain carboxyl group of Asp or Glu residue at the 3-position to effect cyclization. Where the cyclic structure is formed via a disulfide bond, Ala residue at the 8-position of HCNP is firstly replaced with Cys residue, Ala residue at the 2-position or Ile residue at the 4-position is then replaced with Cys residue and, a disulfide bond is formed between the side chain thiol group of Cys residue at the 8-position and the side chain thiol group of Cys residue at the 2-

or 4-position to effect cyclization.

Regarding the fragmentation, it is selected from fragmentation for reducing amino acid residues from the N-terminus of HCNP, fragmentation for reducing amino acid residues from the C-terminus and fragmentation for reducing amino acid residues from both N-terminus and C-terminus. The fragmentation is characterized by containing the dipepetide portion at the 6- and 7-positions as the minimum fragment.

Representative examples of preferred fragments are illustrated below.

Ala-Ala-Asp-Ile-Ser-Gln-Trp-Ala;

Ala-Asp-Ile-Ser-Gln-Trp-Ala;

Ala-Ala-Asp-Ile-Ser-Gln-Trp;

Ala-Asp-Ile-Ser-Gln-Trp-Ala-Gly-Pro-Leu;

Ile-Ser-Gln-Trp-Ala;

Gln-Trp;

Ala-Asp-Ile-Ser-Gln-Trp; and

Gln-Trp-Ala-Gly-Pro-Leu.

The N-terminal modification comprises converting the N-terminal structure ($CH_3CO$- or H-) of HCNP into other structures. The "other structures" as used herein mean various acyl groups other than acetyl group, various sulfonyl groups and various residues necessary to forming a urea skeleton or a urethane skeleton together with the amino group of N-terminal amino acid residue. Herein the N-terminal modification is not the structural conversion mandatorily required for the present invention and hence, X in formula I which shows the N-terminal structure of the neurotrophic peptide derivatives also includes $CH_3CO$- and H- that are the N-terminal structures of HCNP, namely, X refers to H, an acyl group, sulfonyl group or a residue for forming a urea skeleton or a urethane skeleton together with the amino group of amino acid residue to which X is bound.

Representative examples of X include H;

$$\begin{array}{c} R^1a \\ | \\ R^1b - C - CO - \\ | \\ R^1c \end{array}$$

[wherein $R^1a$ represents H, an unsubstituted or substituted alkyl, hydroxy, $-CO_2H$, aryl, $C_1$-$C_4$ alkoxy, halo, $-CONR^2R^3$ [wherein each of $R^2$ and $R^3$ independently represents H or $C_1$-$C_4$ alkyl] or a heterocyclic group; $R^1b$ represents H, an unsubstituted or substituted alkyl or halo; and $R^1c$ represents H, $C_1$-$C_4$ alkyl or halo];

$$\begin{array}{c} R^4a - N - CO - \\ | \\ R^4b \end{array}$$

[wherein $R^4a$ represents H, $C_1$-$C_4$ alkyl or aryl, and $R^4b$ represents H or $C_1$-$C_4$ alkyl];

$R^5$ - O - CO -

[wherein $R^5$ represents an unsubstituted or substituted alkyl or aryl];

$R^6$ - CO -

[wherein $R^6$ is H, aryl or heterocyclic group];

$R^7$ -$SO_2$ -

[wherein $R^7$ is unsubstituted or substituted alkyl or aryl]; and the like.

The C-terminal modification comprises converting the C-terminal structure (-OH) of HCNP into other structures. The "other structures" as used herein mean various residues for forming an amide group or an ester group together with the carbonyl group of C-terminal amino acid residue. Herein the C-terminal modification is not the structural conversion mandatorily required for the present invention and hence, Y in formula I which shows the C-terminal structure of the neurotrophic peptide derivatives also includes -OH that is the C-terminal structure of HCNP, namely, Y refers to OH or a residue for forming an amide group or an ester group together with the carbonyl group of amino acid residue to which Y is bound.

Representative examples of Y include:

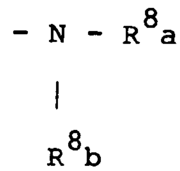

[wherein $R^8$a is H, unsubstituted or substituted alkyl, hydroxy, aryl or heterocyclic group; and $R^8$b is H or unsubstituted or substituted alkyl];

- O - $R^9$

[wherein $R^9$ is H, unsubstituted or substituted alkyl, aryl or heterocyclic group];

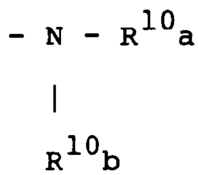

[wherein $R^{10}$a and $R^{10}$b are combined to form a nitrogen-containing saturated heterocyclic group together with N]; and the like.

More particular example of Y is $NH_2$, $NH(CH_2)_nNH_2$, $NH(CH_2)_nN(CH_3)_2$ or $NH(CH_2)_nNHC(=NH)NH_2$ (wherein n represents an integer of 2 to 8).

Hereafter representative examples of the neurotrophic peptide derivatives of the present invention are given below.

H-D-Ala-Ala-Asp-Ile-Ser-Gln-Trp-Ala-Gly-Pro-Leu-OH

Ac-Tyr-Ala-Asp-Ile-Ser-Gln-Trp-Ala-Gly-Pro-Leu-OH

H-Arg-Ala-Asp-Ile-Ser-Gln-Trp-$NH(CH_2)_8NH_2$

H-MeAla-Ala-Asp-Ile-Ser-Gln-Trp-$NH(CH_2)_8NH_2$

H-Ala-Met-Asp-Ile-Ser-Gln-Trp-Ala-Gly-Pro-Leu-OH

H-Ala-Met(O)-Asp-Ile-Ser-Gln-Trp-Ala-Gly-Pro-Leu-OH

H-Ala-Met($O_2$)-Asp-Ile-Ser-Gln-Trp-Ala-Gly-Pro-Leu-OH

H-Ala-Ala-Gln-Ile-Ser-Gln-Trp-Ala-Gly-Pro-Leu-OH

H-Ala-Asp-Pro-Ser-Gln-Trp-Ala-Gly-Pro-Leu-OH

H-Ala-Asp-Aib-Ser-Gln-Trp-Ala-Gly-Pro-Leu-OH

H-MeIle-Ser-Gln-Trp-Ala-$NH_2$

pGlu-Trp-OH

H-Ala-Ala-Asp-Ile-Ser-Gln-D-Trp-Ala-Gly-Pro-Leu-OH

H-Ile-Ser-Gln-D-Trp-Ala-$NH_2$

H-MeIle-Ser-Gln-D-Trp-Ala-$NH(CH_2)_3NH_2$

H-MeIle-Ser-Gln-D-Trp-Ala-$NH(CH_2)_3N(CH_3)_2$

H-MeIle-Ser-Gln-D-Trp-Ala-$NH(CH_2)_4NHC(=NH)NH_2$

$Me_2$Ile-Ser-Gln-D-Trp-Ala-$NH(CH_2)_8NH_2$

$Me_3$Ile-Ser-Gln-D-Trp-Ala-$NH(CH_2)_8NH_2$

AIle-Ser-Gln-D-Trp-Ala-OH

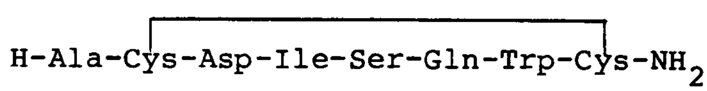

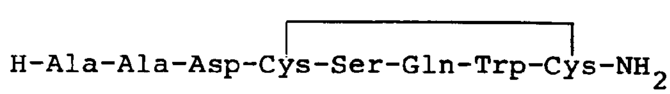

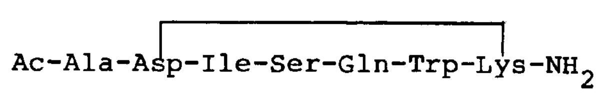

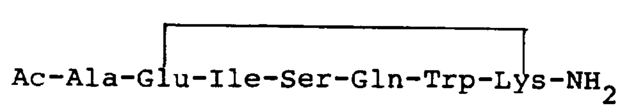

Where the abbreviation HCNP is used to simply refer to a structure of derivative, the abbreviation HCNP

means:

H-Ala-Ala-Asp-Ile-Ser-Gln-Trp-Ala-Gly-Pro-Leu-OH.

The term "alkyl" used in the present invention means a branched and straight saturated aliphatic hydrocarbon group having a specific number of carbon atoms. For example, $C_1$-$C_4$ alkyl means methyl, ethyl, propyl, butyl, isopropyl, isobutyl, t-butyl, etc. The term "alkoxy" means an alkyl group having a specific number of carbon atoms which is bound via an oxygen atom. For example, $C_1$-$C_4$ alkoxy means methoxy, ethoxy, propoxy, butoxy, etc. The term "halo" means fluoro, chloro, bromo and iodo. The term "aryl" is used to mean phenyl, naphthyl or anthryl, etc. which may optionally be substituted with 1 to 3 groups independently selected from the group consisting of: $C_1$-$C_8$ alkyl, amino, mono- or di-$C_1$-$C_4$ alkylamino, amino-$C_1$-$C_8$ alkyl, mono- or di-$C_1$-$C_4$ alkylamino-$C_1$-$C_8$ alkyl, guanidino, $C_1$-$C_4$ alkylguanidino, guanidino-$C_1$-$C_8$ alkyl, $C_1$-$C_4$ alkylguanidino-$C_1$-$C_8$ alkyl, hydroxyl, hydroxy-$C_1$-$C_8$ alkyl, $C_1$-$C_4$ alkoxy, -$CO_2H$, carboxy-$C_1$-$C_8$ alkyl, halo, $NO_2$, $CF_3$ and -$CONR^2R^3$ [$R^2$ and $R^3$ have the same significances as described above] and the like. The term "$C_1$-$C_4$ alkylguanidino" means a guanidino group in which the guanidino nitrogen atom is alkylated by one or two $C_1$-$C_4$ alkyl groups. The term "heterocyclic group" is used to mean a saturated or unsaturated 3-to 8-membered monocyclic or 9- to 10-membered fused heterocyclic group. The heterocyclic group is composed of carbon atoms and 1 to 3 hetero atoms selected from the group consisting of N, O and S. The nitrogen and sulfur hetero atoms may optionally be oxidized; or the nitrogen hetero atom may optionally be quaternized. The binding site is on any of the carbon atoms but with respect to $R^1$a, in the case of a hetero ring containing at least one nitrogen atom, the nitrogen atom can be the binding site.

Examples of such saturated heterocyclic group include pyrrolidinyl, piperidyl, piperidino, homopiperidyl, heptamethyleneiminyl, piperazinyl, homopiperazinyl, morpholinyl, morpholino, thioranyl, thiomorpholinyl, thiomorpholinylsufoxide, thiomorpholinylsulfone, tetrahydrofuryl, etc. Such saturated heterocyclic moieties may also be optionally substituted with 1 or 2 groups independently selected from the group consisting of: hydroxy, carboxyl, carboxyl-$C_1$-$C_8$ alkyl, aryl, aryl-$C_1$-$C_4$ alkyl, $C_1$-$C_4$ alkyl, amino, mono- or di-$C_1$-$C_4$ alkylamino, amino-$C_1$-$C_8$ alkyl, mono-or di-$C_1$-$C_4$ alkylamino-$C_1$-$C_8$ alkyl, hydroxy-$C_1$-$C_4$ alkyl, guanidino, $C_1$-$C_4$ alkylguanidino, guanidino-$C_1$-$C_8$ alkyl, $C_1$-$C_4$ alkylguanidino-$C_1$-$C_8$ alkyl, -$N(R^{11})_3A$ [wherein $R^{11}$ represents $C_1$-$C_4$ alkyl and A represents a counter ion selected from the group consisting of monovalent anions], $N(R^{11})_3A$-substituted $C_1$-$C_8$ alkyl [wherein $R^{11}$ and A have the same significances as described above], and the like. Examples of such unsaturated heterocyclic groups include pyrrolyl, pyridyl, pyrazinyl, imidazolyl, pyrazolyl, furyl, oxazolyl, thienyl, thiazolyl, indolyl, quinolyl, isoquinolyl, etc. Such unsaturated heterocyclic group may optionally be substituted with a group selected from the group consisting of $CF_3$, $C_1$-$C_4$ alkyl, $C_1$-$C_4$ alkoxy, halo, etc. The term "counter ion" means monovalent anion such as chloride, bromide, acetate, perchlorate, benzoate, maleate, benzenesulfonate, tartarate, hemitartarate, etc. The term "nitrogen-containing saturated heterocyclic group" means a saturated 3- to 8-membered monocyclic nitrogen-containing heterocyclic group, which is composed of at least one nitrogen atom, carbon atoms and, if necessary, one hetero atom selected from the group consisting of O and S and which binding site is on the nitrogen atom. The nitrogen and sulfur hetero atoms may optionally be oxidized or the nitrogen atom may also be quaternized. Examples of such nitrogen-containing saturated heterocyclic group include pyrrolidinyl, piperidino, homopiperidino, heptamethyleneiminyl, piperazinyl, homopiperazinyl, morpholino, thiomorpholino, thiomorpholinosulfoxide, thiomorpholinosulfone, etc. Such nitrogen-containing saturated heterocyclic groups may also be optionally substituted with 1 or 2 groups independently selected from the group consisting of: hydroxy, carboxyl, carboxyl-$C_1$-$C_8$ alkyl, aryl, aryl-$C_1$-$C_4$ alkyl, $C_1$-$C_4$ alkyl, amino, mono- or di-$C_1$-$C_4$ alkylamino, amino-$C_1$-$C_8$ alkyl, mono- or di-$C_1$-$C_4$ alkylamino-$C_1$-$C_8$ alkyl, hydroxy-$C_1$-$C_4$ alkyl, guanidino, $C_1$-$C_4$ alkylguanidino, guanidino-$C_1$-$C_8$ alkyl, $C_1$-$C_4$ alkylguanidino-$C_1$-$C_8$ alkyl, -$N(R^{11})_3A$ [wherein $R^{11}$ represents $C_1$-$C_4$ alkyl and A represents a counter ion selected from the group consisting of monovalent anions], $N(R^{11})_3A$-substituted $C_1$-$C_8$ alkyl [wherein $R^{11}$ and A have the same significances as described above], and the like. The term "unsubstituted or substituted alkyl" means an unsubstituted branched and straight saturated aliphatic hydrocarbon group having 1 to 16 carbon atoms, i.e., $C_1$-$C_{16}$ alkyl, which may optionally be substituted with a group selected from the group consisting of: amino, mono- or di-$C_1$-$C_4$ alkylamino, hydroxy, -$CO_2H$, guanidino, $C_1$-$C_4$ alkylguanidino, aryl, $C_1$-$C_4$ alkoxy, halo, -$N(R^{11})_3A$ [wherein $R^{11}$ and A have the same significances as described above], -$CONR^2R^3$ [wherein $R^2$ and $R^3$ have the same significances as described above] and a heterocyclic group [in the case of a heterocyclic group containing at least one nitrogen atom, the nitrogen atom can be the binding site].

In the specification, amino acids, protective groups, active groups, solvents and the like are sometimes referred to by their abbreviations based on IUPAC-IUB and abbreviations conventionally used in the art. The abbreviations for amino acid residues or amino acid derivatives are shown below.

| Abbreviations | Name (structure) |
|---|---|
| Ala | Alanine |
| MeAla | N-Methylalanine |
| Cys | Cysteine |
| Cys Cys | Cystine |
| Asp | Aspartic acid |
| Asp Lys | ε-(ß-Aspartyl)-lysine |
| Glu | Glutamic acid |
| Glu Lys | ε-(γ-Glutamyl)-lysine |
| pGlu | Pyroglutamic acid |
| Gly | Glycine |
| Ile | Isoleucine |
| MeIle | N-Methylisoleucine |
| $Me_2Ile$ | N-Dimethylisoleucine |
| $Me_3Ile$ | N-Trimethylisoleucine |
| AIle | N-Amidinoisoleucine 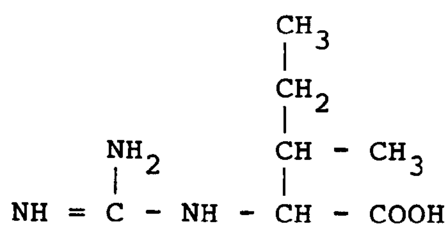 |
| Lys | Lysine |
| Leu | Leucine |
| Met | Methionine |
| Met(O) | Methionine sulfoxide |
| $Met(O_2)$ | Methionine sulfone |

| | |
|---|---|
| Pro | Proline |
| Gln | Glutamine |
| Arg | Arginine |
| Ser | Serine |
| Trp | Tryptophan |
| Tyr | Tyrosine |
| Asx | Asparagine or Aspartic acid |
| Glx | Glutamine or Glutamic acid |
| Aib | 2-Aminoisobutyric acid |

Unless otherwise indicated, the amino acid residues given without the prefix "L" in the specification correspond to the naturally occurring absolute configuration L.

Other abbreviations are shown below.

| Abbreviations | Name |
|---|---|
| Ac | Acetyl |
| Boc | t-Butyloxycarbonyl |
| $(Boc)_2O$ | di-t-Butyldicarbonate |
| Z | Benzyloxycarbonyl |
| Z-Cl | Benzyloxycarbonyl chloride |
| Tos | p-Toluenesulfonyl |
| OcHex | Cyclohexyl ester |
| Bzl | Benzyl |
| 4-MeBzl | 4-Methylbenzyl |

| | |
|---|---|
| $Cl_2$-Bzl | 2,6-Dichlorobenzyl |
| CHO | Formyl |
| DCC | Dicyclohexylcarbodiimide |
| EDC | 1-Ethyl-3-(3-dimethylamino-propyl)-carbodiimide |
| EDC.HCl | 1-Ethyl-3-(3-dimethylamino-propyl)-carbodiimide hydrochloride |
| IBCF | Isobutyroxycarbonyl chloride |
| DMF | Dimethylformamide |
| NMP | N-Methyl-2-pyrrolidinone |
| AcOEt | Ethyl acetate |
| TFA | Trifluoroacetic acid |
| TEA | Triethylamine |
| MBHA | 4-Methylbenzhydrylamine |
| HOBt | 1-Hydroxybenzotriazole |
| HONp | p-Nitrophenol |
| PTC | Phenylthiocarbamyl |
| ACh | Acetylcholine |

The pharmaceutically acceptable salts of the neurotrophic peptide derivatives of the present invention include conventional non-toxic salts of these peptides and quaternary salts thereof. These salts may be formed from inorganic or organic acids or bases. Examples of such acid addition salts are salts of acetic acid, butyric acid, citric acid, lactic acid, tartaric acid, oxalic acid, maleic acid, succinic acid, fumaric acid, hydrochloric acid, hydrobromic acid and sulfuric acid. Salts of bases are ammonium salts, alkali metal salts such as sodium and potassium salts, alkaline earth metal salts such as calcium and magnesium salts, salts with amino acids such as arginine, lysine, etc. Such salts can be readily produced by known methods. For example, in the case of acetates, the acetates can be prepared by dissolving the neurotrophic peptide derivatives in water and adding a necessary amount of acetic acid to the solution.

[Methods for preparation]

The neurotrophic peptide derivatives of the present invention can be synthesized in a manner similar to methods conventionally used in ordinary peptide chemistry. Such known methods are described in, for example, M. Bodansky and M.A. Ondetti, Peptide Synthesis, published by Interscience Publishing Co., New York, 1966; F.M. Finn and K. Hofmann, The Proteins, volume 2, edited by H. Neurath, R.L. Hill, Academic Press Inc., New York, 1976; Nobuo Izumiya et al., Peptide Synthesis, published by Maruzen Co., 1975; Nobuo Izumiya et al., Fundamental Peptide Synthesis and Experiment, published by Maruzen Co., 1985; Lecture Series on

Biochemical Experiment, edited by the Association of Biochemistry, Japan, volume 1, "Chemistry of Protein IV", chapter II, Haruaki Yajima, Peptide Synthesis, 1977; etc. That is, the peptide can be synthesized by selecting any of the liquid phase method and the solid phase method, depending upon the structure of the C-terminus. In more detail, where peptides contain a partial structure of -COOH or $-CONH_2$ at the C-terminus, the peptides can be obtained by any of the liquid phase method and the solid phase method but in other cases, the liquid phase method is rather preferred.

For example, in the case that the peptide derivative is synthesized by the solid phase method, the C-terminal amino acid (amino group-protected amino acid) or the C-terminal substituent (the substituent having carboxyl group; in the case that an amino group is contained, the amino group is protected) is bound to insoluble carrier through the carboxyl group. If necessary and desired, after the amino protective group is removed, the amino group-protected amino acids or the amino acid derivatives (in the case that a free amino group is present, the amino group is protected) are successively coupled, according to the amino acid sequence of the desired peptide, through condensation of the reactive carboxyl groups with the reactive amino groups or with the reactive hydroxy groups. The synthesis is carried out step by step. If necessary, after the selected side chain protective group is removed, cyclization reaction may be carried out. After synthesis of the whole sequence, the N-terminal substituent is condensed, if necessary. Then, the peptide is withdrawn from the insoluble carrier and at the same time, the protective group is removed. Further if necessary and desired, the N-terminal substituent or C-terminal substituent is condensed and the protective group is removed to obtain the desired peptide. Further if necessary, the desired bond is finally formed between the side chains to obtain the cyclic peptide.

In the case of synthesis by the liquid phase method, the C-terminal amino acid having a free amino group at the terminal (carboxyl group-protected amino acid) or the C-terminal substituent (the substituent having free amino or hydroxy group; in the case that a carboxyl group is present, the carboxyl group is protected) is successively coupled with the amino group-protected amino acid or the amino acid derivatives (in the case that a free amino group is present, the amino group is protected), according to the amino acid sequence of the desired peptide, through condensation of the reactive amino groups or the reactive hydroxy groups with the reactive carboxyl groups. If necessary, the N-terminal substituent is finally condensed therewith. Further if necessary, the N-terminal amino acid may be derivatized. Thus, the whole sequence can be synthesized. The whole sequence may also be synthesized by synthesizing in a similar manner, removing the selected protective groups and coupling the resulting peptide fragments to each other. The protective group is removed and if necessary, the N-terminal substituent or the C-terminal substituent is condensed and the protective group is removed to obtain the desired peptide. Moreover, the desired bond is finally formed between the side chains and, if necessary, the protective group is removed to obtain the cyclic peptide.

In the methods described above, the reactive functional groups are preferably protected.

Examples of the protective group of the amino group include benzyloxycarbonyl, t-butyloxycarbonyl, p-methoxybenzyloxycarbonyl, 2-chlorobenzyloxycarbonyl, p-toluenesulfonyl, trifluoroacetyl, phthalyl, formyl, o-nitrophenylsulfenyl, 3-nitro-2-pyridinesulfenyl, diphenylphosphinothioyl, etc. Examples of the protective group of the carboxyl group include alkyl esters (esters of $C_1$-$C_4$ such as methyl, ethyl, t-butyl, etc.), benzyl ester, p-nitrobenzyl ester, p-methylbenzyl ester, cyclohexyl ester, cyclopentyl ester, etc. Examples of the protective group of the mercapto group include benzyl, p-methoxybenzyl, 4-methylbenzyl, trityl, benzhydryl, acetamidomethyl, 3-nitro-2-pyridinesulfenyl, t-butyl, etc. The hydroxy group in Ser, Tyr, etc. may not be necessarily protected but if necessary, can be protected with benzyl, 2,6-dichlorobenzyl, t-butyl, benzyloxycarbonyl, acetyl, etc. The indolyl group in Trp, etc. may be protected, if necessary, with formyl, benzyl-oxycarbonyl, 2,4-dichlorobenzyloxycarbonyl, etc. The guanidino group may also function to be protected in the state protonated with hydrochloric acid, etc. but, if necessary, may also be protected with p-toluene-sulfonyl, nitro, benzyloxycarbonyl, p-methoxybenzenesulfonyl, mesitylene-2-sulfonyl, etc. In the methods described above, peptide bonds can be formed by known methods, for example, the method using condensing agents of carbodiimide type such as dicyclohexyl-carbodiimide, 1-ethyl-3-(3-dimethylaminopropyl)carbodiimide, etc.; the symmetrical acid anhydride method, the mixed acid anhydride method, the azide method, the activated ester method, the oxidation-reduction method, the diphenylphosphoryl azide method, the method using carbodiimide type condensing agent and additives (1-hydroxybenzotriazole, N-hydroxysuccinimide, etc.).

For removing the protective group, there are known, for example, the trifluoroacetic acid method, the methanesulfonic acid method, the trifluoromethanesulfonic acid method, the hydrogen fluoride method, the liquid ammonia-sodium method, the catalytic reduction method, the alkali saponification method, etc.

The peptides produced by the present invention can be purified by using known methods conventionally used in the art of peptide chemistry, singly or in combination, such as ion exchange chromatography, partition chromatography, gel chromatography, reverse phase liquid chromatography, etc.

[Pharmacological activities]

The neurotrophic peptide derivatives of the present invention can regulate differentiation and maturation of neuronal cells. That is, the neurotrophic peptide derivatives accelerate the acetylcholine synthesis in the tissue of medial septum nuclei which is rich in cholinergic neurons. The biological activity can be determined by the method of Ojika, K., et al. [Ojika, K., et al., Japanese Journal of Psycopharmacology, 7, 447-451, (1987)].

[Application to therapeutic compositions]

The neurotrophic peptide derivatives of the present invention are useful for the treatment of neurological degenerative disorder and dementia. The neurological degenerative disorder is a disease caused by degeneration/denervation of cholinergic neuron and is exemplified by Alzheimer syndrome, Alzheimer type dementia, amyotrophic lateral sclerosis, Parkinson's disease, etc. As dementia, there are Alzheimer type dementia, Parkinson's dementia, cerebro-vascular dementia.

Animal to which the neurotrophic peptide derivatives of the present invention are applicable is not limited. The compounds of the present invention can be applied to human beings as well as to other various species of mammals such as mouse, rat, dog, calf, horse, goat, sheep, rabbit, hog, etc.

The neurotrophic peptide derivatives of the present invention can be administered to these animals and human by ordinary route, for example, orally, intramuscularly, intravenously, subcutaneously, intraperitoneally, pernasally and intracerebrally. Dose and time of administration vary depending upon animal species, administration route, condition of disease, body weight, etc. In human, the peptides can be administered to adult in a daily dose of approximately 1 μg to 1 g, once or in several portions. Examples of pharmceutical preparations include powders, granulates, granules, tablets, capsules, suppositories, injections, nasal preparations, etc. The pharmaceutical preparations can be prepared in a conventional manner, using conventional carriers for preparations. That is, in the case of preparing oral preparations, excipients or carriers are added to the active ingredient and if necessary, binders, disintegrators, lubricants and coloring agents, are further added thereto and then prepared into tablets, granules, powders, capsules, etc., by known methods. In the case of preparing injections, pH regulators, buffers, stabilizers, solubilizing agents, etc. are added depending upon necessity and prepared into injections in a conventional manner.

Hereafter the present invention is described in more detail by referring to the examples below.

Example 1

Synthesis of [D-$Ala^1$]HCNP:

Chloromethylated polystyrene vinylbenzene resin (crosslinked with 1% divinylbenzene, containing 0.68 mmol of chloride per g of the resin) of 100 to 200 mesh was employed. Upon synthesis of [D-$Ala^1$]HCNP, 4.07 g of Boc-Leu-OH was dissolved in a mixture of 30 ml of ethyl alcohol and 10 ml of water and pH was adjusted to 7 with 20% cesium carbonate solution. The solution was concentrated in vacuo and desiccated. To the residue was added 160 ml of DMF and further added 20 g of the chloromethylated resin. The mixture was stirred at 50°C for 12 hours and then at room temperature for further 12 hours to esterify. The resulting Boc-Leu-O-resin was filtered, washed sequentially with DMF, 90% DMF, DMF and ethyl alcohol and then desiccated. Yield, 23.4 g.

Twenty grams of this Boc-Leu-O-resin were charged in a solid phase synthesis reactor. Following Schedule 1 described hereinafter, Boc-Pro-OH, Boc-Gly-OH, Boc-Ala-OH, Boc-Trp-OH, Boc-Gln-OH and Boc-Ser(Bzl)-OH were successively coupled with the resin. During the course of the reaction, a part of the resin after the coupling of Boc-Trp-OH and Boc-Gln-OH was completed was taken out. Finally, 24.50 g of HCNP(5-11) peptide resin was obtained. Following Schedule 2 later described, 1.00 g of this HCNP(5-11) resin was coupled sequentially with Boc-Ile-OH, Boc-Asp(OcHex)-OH, Boc-Ala-OH and Boc-D-Ala-OH. As the result, 1.22 g of [D-$Ala^1$]HCNP peptide resin was obtained.

To 0.61 g of this [D-$Ala^1$]HCNP peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was dissolved in 200 ml of 2.5% acetic acid aqueous solution. The solution was applied to reverse phase YMC-ODS-A120-S-10/20 column (30 Ø x 300 mm) previously equilibrated with 0.1% TFA aqueous solution. After

the column was washed with 0.1% TFA aqueous solution, the peptide was eluted with linear gradient of 0 to about 25% acetonitrile in 200 minutes at a flow rate of 6.0 ml/min. The eluent was monitored at A280 nm. The fractions containing the desired product were collected and lyophilized to give 49.3 mg of [D-$Ala^1$]HCNP.

The thus obtained [D-$Ala^1$]HCNP was eluted at retention time of 26.5 minutes with linear density gradient of 10-50% aqueous acetonitrile containing 0.1% TFA through reverse phase YMC-AM303(S-5)-ODS column (4.6 Ø x 250 mm). The amino acid analysis of the peptide coincided with the calculated values.

Amino acid analysis

Hydrolysis: 4N Methanesulfonic acid, 2% tryptamine, at 110°C for 24 hours

Analysis method: PICO-TAG (reverse phase-PTC amino acid) method

Result:

| | |
|---|---|
| Asx: | 1.09 (1) |
| Glx: | 1.06 (1) |
| Ser: | 0.96 (1) |
| Gly: | 1.07 (1) |
| *Ala: | 3.00 (3) |
| Pro: | 1.07 (1) |
| Ile: | 1.05 (1) |
| Leu: | 1.06 (1) |
| Trp: | 0.86 (1) |

*Ala was used as a standard amino acid. The values in parentheses indicate calculated values.

Schedule 1

| | Steps | Time (min.) x Treatment times |
|---|---|---|
| 1. | Washing with methylene chloride, 200 ml | 2 x 3 |
| 2. | Deprotection with 50% TFA, 5% ethanedithiol, 45% methylene chloride (V/V), 200 ml | 3 x 1<br>20 x 1 |
| 3. | Washing with methylene chloride, 200 ml | 2 x 2 |
| 4. | Washing with methanol, 200 ml | 2 x 2 |
| 5. | Neutralization with 10% triethylamine, 90% methylene chloride (V/V), 200 ml | 1 x 1 |
| 6. | Washing with methanol, 200 ml | 2 x 1 |
| 7. | Neutralization with 10% triethylamine, 90% methylene chloride (V/V), 200 ml | 1 x 1 |
| 8. | Washing with methanol, 200 ml | 2 x 2 |
| 9. | Washing with methylene chloride, 200 ml | 2 x 3 |
| 10. | Coupling by the use of various amino group-protected amino acids (20 mmols), additive (HOBt) 50% DMF-50% methylene chloride (V/V), 100 ml | 5 x 1 |
| | Solution of DCC (20 mmols) in methylene chloride, 40 ml | 120 x 1 |
| 11. | Washing with 50% DMF, 50% methylene chloride (V/V), 200 ml | 2 x 2 |
| 12. | Washing with methanol, 200 ml | 2 x 1 |
| 13. | Neutralization with 10% triethylamine, 90% methylene chloride (V/V), 200 ml | 1 x 1 |
| 14. | Washing with methanol, 200 ml | 2 x 2 |

| | | |
|---|---|---|
| 15. | Washing with methylene chloride, 200 ml | 2 x 2 |
| 16. | Acetylation with 25% acetic anhydride, 75% methylene chloride (V/V), 200 ml | 15 x 1 |
| 17. | Washing with methylene chloride 200 ml | 2 x 2 |
| 18. | Washing with methanol, 200 ml | 2 x 2 |

After the coupling reaction in the step 10, where a small amount of the resin was subjected to ninhydrin test to show positive blue indicative of incompleteness of the coupling reaction, the coupling reaction was repeated using an amino acid of the same protection type.

Schedule 2

| | Steps | Time (min.) x Treatment times |
|---|---|---|
| 1. | Washing with methylene chloride, 30 ml | 2 x 3 |
| 2. | Deprotection with 50% TFA, 5% ethanedithiol, 45% methylene chloride (V/V), 30 ml | 3 x 1<br>20 x 1 |
| 3. | Washing with methylene chloride, 30 ml | 2 x 2 |
| 4. | Washing with methanol, 30 ml | 2 x 2 |
| 5. | Neutralization with 10% triethylamine, 90% methylene chloride (V/V), 30 ml | 1 x 1 |
| 6. | Washing with methanol, 30 ml | 2 x 1 |
| 7. | Neutralization with 10% triethylamine, 90% methylene chloride (V/V), 30 ml | 1 x 1 |

| | | |
|---|---|---|
| 8. | Washing with methanol, 30 ml | 2 x 2 |
| 9. | Washing with methylene chloride, 30 ml | 2 x 3 |
| 10. | Coupling by the use of various amino group-protected amino acids (3 mmols), additive (HOBt or HONp) 50% DMF-50% methylene chloride (V/V), 15 ml | 5 x 1 |
| | Solution of DCC (3 mmols) in methylene chloride, 6 ml | 120 x 1 |
| 11. | Washing with 50% DMF, 50% methylene chloride (V/V), 30 ml | 2 x 2 |
| 12. | Washing with methanol, 30 ml | 2 x 1 |
| 13. | Neutralization with 10% triethylamine, 90% methylene chloride (V/V), 30 ml | 1 x 1 |
| 14. | Washing with methanol, 30 ml | 2 x 2 |
| 15. | Washing with methylene chloride, 30 ml | 2 x 2 |
| 16. | Acetylation with 25% acetic anhydride, 75% methylene chloride (V/V), 30 ml | 15 x 1 |
| 17. | Washing with methylene chloride 30 ml | 2 x 2 |
| 18. | Washing with methanol, 30 ml | 2 x 2 |

After the coupling reaction in the step 10, where a small amount of the resin was subjected to ninhydrin test to show positive blue indicative of incompleteness of the coupling reaction, the coupling reaction was repeated using an amino acid of the same protection type. In the case of coupling subsequent to twice occurrences, DMF or 1-methyl-2-pyrrolidinone was used instead of 50% DMF-50% methylene chloride (V/V) and the coupling reaction was carried out for a maximum of 12 hours.

Example 2

Synthesis of Ac[$Tyr^1$]HCNP:

In a solid phase synthesis reactor was charged 6.0 g of HCNP(5-11) peptide resin described in Example 1. Following Schedule 3 described hereinafter, Boc-Ile-OH, Boc-Asp(OcHex)-OH and Boc-Ala-OH were successively coupled with the peptide resin. During the course of the reaction, a part of the resin was taken out at the time when the coupling was completed. Finally, 3.70 g of HCNP(2-11) peptide resin was obtained. Following Schedule 2 described in Example 1, 1.50 g of this HCNP(2-11) peptide resin was coupled with Boc-Tyr($Cl_2$Bzl)-OH. A part of the resin was taken out and the remaining resin was further subjected to deprotection and acetylation by repeating steps 1 through 9 and then steps 16 to 18 of Schedule 2. As the result, 0.94 g of Aq[$Tyr^1$]HCNP peptide resin was obtained.

To 0.94 g of this Ac[Tyr[1]]HCNP peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 60 minutes, filtered and washed with 100 ml of diethyl ether. To the residue was added 100 ml of 20% acetic acid aqueous solution. After stirring for 30 minutes, the resin was filtered off and washed with 130 ml of 20% acetic acid aqueous solution. After the filtrate was diluted with 230 ml of water, the dilution was applied to reverse phase YMC-A-363(S-5)ODS column (30 ø x 250 mm) previously equilibrated with 0.1% TFA aqueous solution. After the column was washed with 0.1% TFA aqueous solution, the peptide was eluted with linear gradient of 0 to about 35% acetonitrile in 180 minutes at a flow rate of 7.0 ml/min. The eluent was monitored at A280 nm. The fractions containing the desired product were collected and lyophilized to give 151.6 mg of Ac[Tyr[1]]HCNP.

The thus obtained Ac[Tyr[1]]HCNP was eluted at retention time of 31.3 minutes with linear density gradient of 10-50% aqueous acetonitrile containing 0.1% TFA through reverse phase YMC-AM303(S-5)-ODS column (4.6 ø x 250 mm). The amino acid analysis of the peptide coincided with the calculated values.

Amino acid analysis

Hydrolysis: 4N Methanesulfonic acid, 2% tryptamine, at 110°C for 24 hours

Analysis method: PICO-TAG (reverse phase-PTC amino acid) method

Result:
Asx: 0.94 (1)
Glx: 0.94 (1)
Ser: 0.89 (1)
Gly: 0.96 (1)
Ala: 1.87 (2)
Pro: 0.94 (1)
Ile: 1.05 (1)
*Leu: 1.00 (1)
Trp: 0.58 (1)
Tyr: 1.01 (1)

*Leu was used as a standard amino acid. The values in parentheses indicate calculated values.

Schedule 3

| | Steps | Time (min.) x Treatment times |
|---|---|---|
| 1. | Washing with methylene chloride, 60 ml | 2 x 3 |
| 2. | Deprotection with 50% TFA, 5% ethanediol, 45% methylene chloride (V/V), 60 ml | 3 x 1<br>20 x 1 |
| 3. | Washing with methylene chloride, 60 ml | 2 x 2 |
| 4. | Washing with methanol, 60 ml | 2 x 2 |
| 5. | Neutralization with 10% triethylamine, 90% methylene chloride (V/V), 60 ml | 1 x 1 |
| 6. | Washing with methanol, 60 ml | 2 x 1 |
| 7. | Neutralization with 10% triethylamine, 90% methylene chloride (V/V), 60 ml | 1 x 1 |
| 8. | Washing with methanol, 60 ml | 2 x 2 |
| 9. | Washing with methylene chloride, 60 ml | 2 x 3 |

| | | |
|---|---|---|
| 10. | Coupling by the use of various amino group-protected amino acids (6 mmols), additive (HOBt or HONp) 50% DMF-50% methylene chloride (V/V), 30 ml | 5 x 1 |
| | Solution of DCC (6 mmols) in methylene chloride, 12 ml | 120 x 1 |
| 11. | Washing with 50% DMF, 50% methylene chloride (V/V), 60 ml | 2 x 2 |
| 12. | Washing with methanol, 60 ml | 2 x 1 |
| 13. | Neutralization with 10% triethylamine, 90% methylene chloride (V/V), 60 ml | 1 x 1 |
| 14. | Washing with methanol, 60 ml | 2 x 2 |
| 15. | Washing with methylene chloride, 60 ml | 2 x 2 |
| 16. | Acetylation with 25% acetic anhydride, 75% methylene chloride (V/V), 60 ml | 15 x 1 |
| 17. | Washing with methylene chloride 60 ml | 2 x 2 |
| 18. | Washing with methanol, 60 ml | 2 x 2 |

After the coupling reaction in the step 10, where a small amount of the resin was subjected to ninhydrin test to show positive blue indicative of incompleteness of the coupling reaction, the coupling reaction was repeated using an amino acid of the same protection type. In the case of coupling subsequent to twice occurrences, DMF or 1-methyl-2-pyrrolidinone was used instead of 50% DMF-50% methylene chloride (V/V) and the coupling reaction was carried out for a maximum of 12 hours.

Example 3

Synthesis of [$Arg^1$]HCNP(1-7)$NH(CH_2)_8NH_2$:

(1) Synthesis of HCl.$H_2N(CH_2)_8$NH-Z

After 20.0 g of $H_2N(CH_2)_8NH_2$ was dissolved in 1 liter of acetonitrile, 139 ml of 1 N sodium hydroxide aqueous solution was added to the solution under ice cooling and 22.2 ml of Z-Cl was then dropwise added to the mixture. The mixture was stirred for 3 hours. After the reaction solution was neutralized with 1 N hydrochloric acid, the precipitated crystals were filtered off. The filtrate was further concentrated in vacuo to distill off acetonitrile. The precipitated crystals were filtered, washed with water and then desiccated to give 7.52 g of HCl.$H_2N(CH_2)_8$NH-Z.

(2) Synthesis of Boc-Trp-$NH(CH_2)_8$NH-Z

After 1.0 g of HCl.$H_2N(CH_2)_8$NH-Z was suspended in 20 ml of DMF, 0.37 g of TEA was added to the suspension to neutralize and dissolve. Then 0.50 g of HOBt and 1.1 g of Boc-Trp-OH were added to the solution to dissolve them. Under ice cooling, 0.70 g of EDC.HCl was added to the solution, which was stirred for 30 minutes and then at room temperature for further 5 hours. To the reaction solution was added 200 ml of AcOEt. After washing sequentially with 5% citric acid aqueous solution, saturated sodium hydrogencarbonate aqueous

solution and saturated sodium chloride aqueous solution, the reaction solution was desiccated over magnesium sulfate and the solvent was distilled off to give 1.94 g of Boc-Trp-NH($CH_2$)$_8$NH-Z.

(3) Synthesis of Boc-Gln-Trp-NH($CH_2$)$_8$NH-Z

After 1.94 g of Boc-Trp-NH($CH_2$)$_8$NH-Z was dissolved in 20 ml of acetonitrile, 2.48 g of methanesulfonic acid was added to the solution under ice cooling. The temperature was elevated to room temperature. After stirring for an hour, 20 ml of DMF was added to the mixture. The resulting mixture was neutralized with 2.61 g of TEA under ice cooling. To the solution were added 0.54 g of HOBt and 0.98 g of Boc-Gln-OH to dissolve them. Thereafter, 0.76 g of EDC.HCl was added to the solution, which was stirred for 30 minutes and then at room temperature for further 5 hours. To the reaction solution was added 200 ml of AcOEt. After washing sequentially with 5% citric acid aqueous solution, saturated sodium hydrogencarbonate aqueous solution and saturated sodium chloride aqueous solution, the reaction solution was desiccated over magnesium sulfate and the solvent was distilled off to give 2.05 g of Boc-Gln-Trp-NH($CH_2$)$_8$NH-Z.

(4) Synthesis of Boc-Ser(Bzl)-Gln-Trp-NH($CH_2$)$_8$NH-Z

After 2.05 g of Boc-Gln-Trp-NH($CH_2$)$_8$NH-Z was dissolved in 20 ml of acetonitrile, 3.00 g of methanesulfonic acid was added to the solution under ice cooling. The temperature was elevated to room temperature. After stirring for 3 hours, 20 ml of DMF was added to the mixture. The resulting mixture was neutralized with 3.16 g of TEA under ice cooling. To the solution were added 0.46 g of HOBt and 1.01 g of Boc-Ser(Bzl)-OH to dissolve them. Thereafter, 0.65 g of EDC.HCl was added to the solution, which was stirred for 30 minutes and then at room temperature for further 2 hours. The reaction solution was dropwise added to 150 ml of saturated sodium chloride aqueous solution and the formed precipitates were filtered off. The precipitates were washed 3 times with 50 ml of hexane and then desiccated to give 2.29 g of Boc-Ser(Bzl)-Gln-Trp-NH($CH_2$)$_8$NH-Z.

(5) Synthesis of Boc-Ile-Ser(Bzl)-Gln-Trp-NH($CH_2$)$_8$NH-Z

After 2.29 g of Boc-Ser(Bzl)-Gln-Trp-NH-($CH_2$)$_8$NH-Z was dissolved in 20 ml of acetonitrile, 2.02 g of methanesulfonic acid was added to the solution under ice cooling. The temperature was elevated to room temperature. After stirring for an hour, 20 ml of DMF was added to the mixture. The resulting mixture was neutralized with 2.12 g of TEA under ice cooling. To the solution were added 0.44 g of HOBt and 0.77 g of Boc-Ile-OH to dissolve them. Thereafter, 0.61 g of EDC.HCl was added to the solution, which was stirred for 30 minutes and then at room temperature for further 2 hours. The reaction solution was dropwise added to 150 ml of saturated sodium chloride aqueous solution and the formed precipitates were filtered off. The precipitates were washed 3 times with 50 ml of hexane and then desiccated to give 2.38 g of Boc-Ile-Ser(Bzl)-Gln-Trp-NH($CH_2$)$_8$NH-Z.

(6) Synthesis of Boc-Asp(OcHex)-Ile-Ser(Bzl)-Gln-Trp-NH($CH_2$)$_8$NH-Z

After 2.38 g of Boc-Ile-Ser(Bzl)-Gln-Trp-NH($CH_2$)$_8$NH-Z was dissolved in 20 ml of acetonitrile, 2.71 g of methanesulfonic acid was added to the solution under ice cooling. The temperature was elevated to room temperature. After stirring for 2 hours, 20 ml of DMF and 20 ml of NMP were added to the mixture. The resulting mixture was neutralized with 2.91 g of TEA under ice cooling. To the solution were added 0.36 g of HOBt and 0.84 g of Boc-Asp(OcHex)-OH to dissolve them. Thereafter, 0.51 g of EDC.HCl was added to the solution, which was stirred for 30 minutes and then at room temperature for further 2 hours. The reaction solution was dropwise added to 150 ml of saturated sodium chloride aqueous solution and the formed precipitates were filtered off. The precipitates were washed 3 times with 50 ml of hexane and then desiccated to give 2.80 g of Boc-Asp(OcHex)-Ile-Ser(Bzl)-Gln-Trp-NH($CH_2$)$_8$NH-Z.

(7) Synthesis of Boc-Ala-Asp(OcHex)-Ile-Ser(Bzl)-Gln-Trp-NH($CH_2$)$_8$NH-Z

After 0.60 g of Boc-Asp(OcHex)-Ile-Ser(-Bzl)-Gln-Trp-NH($CH_2$)$_8$NH-Z was dissolved in 10 ml of acetonitrile, 0.74 g of methanesulfonic acid was added to the solution under ice cooling. The temperature was elevated to room temperature. After stirring for 1.5 hour, 20 ml of DMF and 15 ml of NMP were added to the mixture. The resulting mixture was neutralized with 0.78 g of TEA under ice cooling. To the solution were added 0.08 g of HOBt and 0.11 g of Boc-Ala-OH to dissolve them. Thereafter, 0.11 g of EDC.HCl was added to the solution, which was stirred for 30 minutes and then at room temperature for further 3 hours. The reaction solution was dropwise added to 150 ml of saturated sodium chloride aqueous solution and the formed precipitates were filtered off. The precipitates were washed 3 times with 50 ml of hexane and then desiccated to give 0.71 g of Boc-Ala- Asp-(OcHex)-Ile-Ser(Bzl)-Gln-Trp-$NH(CH_2)_8NH$-Z.

(8) Synthesis of Boc-Arg(Tos)-Ala-Asp(OcHex)-Ile-Ser(Bzl)-Gln-Trp-$NH(CH_2)_8NH$-Z

After 0.35 g of Boc-Ala-Asp(OcHex)-Ile-Ser(Bzl)-Gln-Trp-$NH(CH_2)_8NH$-Z was dissolved in 10 ml of acetonitrile, 0.37 g of methanesulfonic acid was added to the solution under ice cooling. The temperature was elevated to room temperature. After stirring for 1.5 hour, 10 ml of DMF and 8 ml of NMP were added to the mixture. The resulting mixture was neutralized with 0.39 g of TEA under ice cooling. To the solution were added 0.04 g of HOBt and 0.12 g of Boc-Arg(Tos)-OH to dissolve them. Thereafter, 0.05 g of EDC.HCl was added to the solution, which was stirred for 30 minutes and then at room temperature for further 3 hours. The reaction solution was dropwise added to 150 ml of saturated sodium chloride aqueous solution and the formed precipitates were filtered off. The precipitates were washed 3 times with 50 ml of hexane and then desiccated to give 0.32 g of Boc-Arg(Tos)-Ala-Asp(OcHex)-Ile-Ser(Bzl)-Gln-Trp-$NH(CH_2)_8NH$-Z.

(9) Synthesis of $[Arg^1]$HCNP(1-7)$NH(CH_2)_8NH_2$

To 0.32 g of Boc-Arg(Tos)-Ala-Asp(OcHex)-Ile-Ser(Bzl)-Gln-Trp-$NH(CH_2)_8NH$-Z were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. The residue was dissolved in 200 ml of 5% acetic acid aqueous solution and the solution was lyophilized. The resulting crude peptide was dissolved in 50 ml of 10% acetic acid aqueous solution and the solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to 21% acetonitrile in 180 minutes and then to 24% in 60 minutes and further to 26% in 60 minutes, at a flow rate of 7.0 ml/min. The eluent was monitored at A280 nm. The fractions containing the desired product were collected and lyophilized to give 17.43 mg of $[Arg^1]$HCNP(1-7)-$NH(CH_2)_8NH_2$.

The thus obtained $[Arg^1]$HCNP(1-7)$NH(CH_2)_8NH_2$ was eluted at retention time of 21.7 minutes with linear density gradient of 10-50% aqueous acetonitrile containing 0.1% TFA through reverse phase YMC-AM303(S-5)-ODS column (4.6 ø x 250 mm). The amino acid analysis of the peptide coincided with the calculated values.

Amino acid analysis

| | |
|---|---|
| Hydrolysis: | 4N Methanesulfonic acid, 2% tryptamine, at 110°C for 24 hours |
| Analysis method: | PICO-TAG (reverse phase-PTC amino acid) method |
| Result: | Asx: 1.05 (1) |
| | Glx: 1.06 (1) |
| | Ser: 0.98 (1) |
| | Arg: 1.04 (1) |
| | *Ala: 1.00 (1) |
| | Ile: 0.99 (1) |
| | Trp: 0.46 (1) |

*Ala was used as a standard amino acid. The values in parentheses indicate calculated values.

Mass spectrum (SIMS)

$[M + H]^+$: 1001.6

Example 4

Synthesis of $[MeAla^1]HCNP(1\text{-}7)NH(CH_2)_8NH_2$

(1) Synthesis of Boc-MeAla-Ala-Asp(OcHex)-Ile-Ser(Bzl)-Gln-Trp-$NH(CH_2)_8NH$-Z

After 0.25 g of Boc-Ala-Asp(OcHex)-Ile-Ser(Bzl)-Gln-Trp-$NH(CH_2)_8NH$-Z described in Example 3 (7) was dissolved in 8 ml of acetonitrile, 0.29 g of methanesulfonic acid was added to the solution under ice cooling. The temperature was elevated to room temperature. After stirring for 1.5 hour, 8 ml of DMF and 3 ml of NMP were added to the mixture. The resulting mixture was neutralized with 0.31 g of TEA under ice cooling. To the solution were added 0.03 g of HOBt and 0.05 g of Boc-MeAla-OH to dissolve them. Thereafter, 0.04 g of EDC.HCl was added to the solution, which was stirred for 30 minutes and then at room temperature for further a day. The reaction solution was dropwise added to 150 ml of saturated sodium chloride aqueous solution and the formed precipitates were filtered off. The precipitates were washed 3 times with 50 ml of hexane and then desiccated to give 0.22 g of Boc-MeAla-Ala-Asp-(OcHex)-Ile-Ser(Bzl)-Gln-Trp-$NH(CH_2)_8NH$-Z.

(2) Synthesis of $[MeAla^1]HCNP(1\text{-}7)NH(CH_2)_8NH_2$

To 0.22 g of Boc-MeAla-Ala-Asp(OcHex)-Ile-Ser(Bzl)-Gln-Trp-$NH(CH_2)_8NH$-Z were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. The residue was dissolved in 200 ml of 5% acetic acid aqueous solution and the solution was lyophilized. The resulting crude peptide was dissolved in 50 ml of 10% acetic acid aqueous solution and the solution was applied to reverse phase YMC-SH-343-5(S-5)ODS column (20 ø x 250 mm) previously equilibrated

with 0.1% TFA aqueous solution. After the column was washed with 0.1% TFA aqueous solution, the peptide was eluted with linear gradient of 0 to 22% acetonitrile in 180 minutes and then to 25% in 60 minutes and further to 27% in 60 minutes, at a flow rate of 7.0 ml/min. The eluent was monitored at A280 nm. The fractions containing the desired product were collected and lyophilized to give 16.63 mg of [MeAla$^1$]HCNP(1-7)-$NH(CH_2)_8NH_2$.

The thus obtained [MeAla$^1$]HCNP(1-7)NH-$(CH_2)_8NH_2$ was eluted at retention time of 23.0 minutes with linear density gradient of 10-50% aqueous acetonitrile containing 0.1% TFA through reverse phase YMC-AM303(S-5)-ODS column (4.6 ø x 250 mm). The amino acid analysis (provided that MeAla was not detected) of the peptide coincided with the calculated values.

Amino acid analysis

Hydrolysis: 4N Methanesulfonic acid, 2% tryptamine, at 110 °C for 24 hours

Analysis method: PICO-TAG (reverse phase-PTC amino acid) method

Result:

Asx: 1.04 (1)

Glx: 1.05 (1)

Ser: 0.99 (1)

*Ala: 1.00 (1)

Ile: 1.00 (1)

Trp: 0.41 (1)

*Ala was used as a standard amino acid. The values in parentheses indicate calculated values.

Mass spectrum (SIMS)

$[M + H]^+$: 930

Example 5

Synthesis of [Met$^2$]HCNP:

In a solid phase synthesis reactor was charged 0.50 g of HCNP(5-11) peptide resin described in Example 1. Following Schedule 4 described hereinafter, Boc-Ile-OH, Boc-Asp(OcHex)-OH and Boc-Met-OH were successively coupled with the peptide resin. A part of the resin was taken out and the remaining resin was further coupled with Boc-Ala-OH following Schedule 4. As the result, 0.16 g of [Met$^2$]HCNP peptide resin was obtained.

To 0.16 g of this [Met$^2$]HCNP peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. After the filtrate was lyophilized, the resulting crude peptide was dissolved in 50 ml of 10% acetic acid aqueous solution. The solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to about

29% acetonitrile in 180 minutes, to 32% in 60 minutes and further to 34% in 60 minutes, at a flow rate of 7.0 ml/min. The eluent was monitored at A280 nm. The fractions containing the desired product were collected and lyophilized to give 9.6 mg of [$Met^2$]HCNP.

The thus obtained [$Met^2$]HCNP was eluted at retention time of 29.0 minutes with linear density gradient of 10-50% aqueous acetonitrile containing 0.1% TFA through reverse phase YMC-AM303(S-5)-ODS column (4.6 ø x 250 mm). The amino acid analysis of the peptide coincided with the calculated values.

Amino acid analysis

| | |
|---|---|
| Hydrolysis: | 4N Methanesulfonic acid, 2% tryptamine, at 110°C for 24 hours |
| Analysis method: | PICO-TAG (reverse phase-PTC amino acid) method |
| Result: | Asx: 1.04 (1) |
| | Glx: 1.02 (1) |
| | Ser: 0.95 (1) |
| | Gly: 1.04 (1) |
| | *Ala: 2.00 (2) |
| | Pro: 1.06 (1) |
| | Met: 0.93 (1) |
| | Ile: 1.04 (1) |
| | Leu: 1.07 (1) |
| | Trp: 0.74 (1) |

*Ala was used as a standard amino acid. The values in parentheses indicate calculated values.

Schedule 4

| | Steps | Time (min.) x Treatment times |
|---|---|---|
| 1. | Washing with methylene chloride, 10 ml | 2 x 3 |
| 2. | Deprotection with 50% TFA, 50% methylene chloride (V/V), 10 ml | 3 x 1<br>20 x 1 |
| 3. | Washing with methylene chloride, 10 ml | 2 x 2 |

| | | |
|---|---|---|
| 4. | Washing with methanol, 10 ml | 2 x 2 |
| 5. | Neutralization with 10% triethylamine, 90% methylene chloride (V/V), 10 ml | 1 x 1 |
| 6. | Washing with methanol, 10 ml | 2 x 1 |
| 7. | Neutralization with 10% triethylamine, 90% methylene chloride (V/V), 10 ml | 1 x 1 |
| 8. | Washing with methanol, 10 ml | 2 x 2 |
| 9. | Washing with methylene chloride, 10 ml | 2 x 3 |
| 10. | Coupling by the use of various amino group-protected amino acids (1 mmol), additive (HOBt) 50% DMF-50% methylene chloride (V/V), 5 ml | 5 x 1 |
| | Solution of DCC (1 mmol) in methylene chloride, 2 ml | 120 x 1 |
| 11. | Washing with 50% DMF, 50% methylene chloride (V/V), 10 ml | 2 x 2 |
| 12. | Washing with methanol, 10 ml | 2 x 1 |
| 13. | Neutralization with 10% triethylamine, 90% methylene chloride (V/V), 10 ml | 1 x 1 |
| 14. | Washing with methanol, 10 ml | 2 x 2 |
| 15. | Washing with methylene chloride, 10 ml | 2 x 2 |
| 16. | Acetylation with 25% acetic anhydride, 75% methylene chloride (V/V), 10 ml | 15 x 1 |
| 17. | Washing with methylene chloride 10 ml | 2 x 2 |
| 18. | Washing with methanol, 10 ml | 2 x 2 |

After the coupling reaction in the step 10, where a small amount of the resin was subjected to ninhydrin test to show positive blue indicative of incompleteness of the coupling reaction, the coupling reaction was repeated using an amino acid of the same protection type.

Example 6

Synthesis of [Met(O)$^2$]HCNP:

In a solid phase synthesis reactor was charged 0.50 g of HCNP(5-11) peptide resin described in Example 1. Following Schedule 4 described in Example 5, Boc-Ile-OH, Boc-Asp(OcHex)-OH and Boc-Met(O)-OH were successively coupled with the peptide resin. A part of the resin was taken out and the remaining resin was further coupled with Boc-Ala-OH following Schedule 4. As the result, 0.16 g of [Met(O)$^2$]HCNP peptide resin was obtained.

To 0.16 g of this [Met(O)$^2$]HCNP peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. After the filtrate was lyophilized, the resulting crude peptide was dissolved in 50 ml of 10% acetic acid aqueous solution. The solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to about 25% acetonitrile in 180 minutes, to 28% in 60 minutes and further to 30% in 60 minutes, at a flow rate of 7.0 ml/min. The eluent was monitored at A280 nm. The fractions containing the desired product were collected and lyophilized to give 18.8 mg of [Met(O)$^2$]HCNP.

The thus obtained [Met(O)$^2$]HCNP was eluted at retention time of 25.6 minutes with linear density gradient of 10-50% aqueous acetonitrile containing 0.1% TFA through reverse phase YMC-AM303(S-5)-ODS column (4.6 ø x 250 mm). The amino acid analysis of the peptide coincided with the calculated values (provided that Met(O) was recovered as Met).

Amino acid analysis

| | |
|---|---|
| Hydrolysis: | 4N Methanesulfonic acid, 2% tryptamine, at 110°C for 24 hours |
| Analysis method: | PICO-TAG (reverse phase-PTC amino acid) method |
| Result: | Asx: 1.07 (1) |
| | Glx: 1.03 (1) |
| | Ser: 0.96 (1) |
| | Gly: 1.03 (1) |

*Ala: 2.00 (2)

Pro: 1.07 (1)

Met: 0.90 (1)

Ile: 1.04 (1)

Leu: 1.07 (1)

Trp: 0.68 (1)

*Ala was used as a standard amino acid. The values in parentheses indicate calculated values.

Example 7

Synthesis of $[Met(O_2)^2]$HCNP:

In a solid phase synthesis reactor was charged 0.50 g of HCNP(5-11) peptide resin described in Example 1. Following Schedule 4 described in Example 5, Boc-Ile-OH, Boc-Asp(OcHex)-OH and Boc-Met($O_2$)-OH were successively coupled with the resin. A part of the resin was taken out and the remaining resin was further coupled with Boc-Ala-OH following Schedule 4. As the result, 0.17 g of $[Met(O_2)^2]$HCNP peptide resin was obtained.

To 0.17 g of this $[Met(O_2)^2]$HCNP peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. After the filtrate was lyophilized, the resulting crude peptide was dissolved in 50 ml of 10% acetic acid aqueous solution. The solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to about 26% acetonitrile in 180 minutes, to 29% in 60 minutes and further to 31% in 60 minutes, at a flow rate of 7.0 ml/min. The eluent was monitored at A280 nm. The fractions containing the desired product were collected and lyophilized to give 23.0 mg of $[Met(O_2)^2]$HCNP.

The thus obtained $[Met(O_2)^2]$HCNP was eluted at retention time of 27.0 minutes with linear density gradient of 10-50% aqueous acetonitrile containing 0.1% TFA through reverse phase YMC-AM303(S-5)-ODS column (4.6 ø x 250 mm). The amino acid analysis of the peptide coincided with the calculated values.

Amino acid analysis

| | |
|---|---|
| Hydrolysis: | 4N Methanesulfonic acid, 2% tryptamine, at 110°C for 24 hours |
| Analysis method: | PICO-TAG (reverse phase-PTC amino acid) method |

Result: Asx: 1.07 (1)

Glx: 1.03 (1)

Ser: 0.94 (1)

Gly: 1.03 (1)

*Ala: 2.00 (2)

Pro: 1.08 (1)

Met($O_2$): 1.07 (1)

Ile: 0.99 (1)

Leu: 1.02 (1)

Trp: 0.69 (1)

*Ala was used as a standard amino acid. The values in parentheses indicate calculated values.

Example 8

Synthesis of [$Gln^3$]HCNP:

In a solid phase synthesis reactor was charged 0.60 g of HCNP(5-11) peptide resin described in Example 1. Following Schedule 4 described in Example 5, Boc-Ile-OH, Boc-Gln-OH and Boc-Ala-OH were successively coupled with the peptide resin. A part of the resin was taken out and the remaining resin was further coupled with Boc-Ala-OH following Schedule 4. As the result, 0.24 g of [$Gln^3$]HCNP peptide resin was obtained.

To 0.24 g of this [$Gln^3$]HCNP peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. After the filtrate was lyophilized. The resulting crude peptide was dissolved in 50 ml of 10% acetic acid aqueous solution and the solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to about 24% acetonitrile in 180 minutes and then to 27% in 60 minutes and further to 29% in 60 minutes, at a flow rate of 7.0 ml/min. The eluent was monitored at A280 nm. The fractions containing the desired product were collected and lyophilized to give 20.0 mg of [$Gln^3$]HCNP.

The thus obtained [$Gln^3$]HCNP was eluted at retention time of 25.4 minutes with linear density gradient of 10-50% aqueous acetonitrile containing 0.1% TFA through reverse phase YMC-AM303(S-5)-ODS column (4.6 ø x 250 mm). The amino acid analysis of the peptide coincided with the calculated values.

Amino acid analysis

Hydrolysis: 4N Methanesulfonic acid, 2% tryptamine, at 110°C for 24 hours

Analysis method: PICO-TAG (reverse phase-PTC amino acid) method

Result:

Glx: 2.14 (2)

Ser: 0.98 (1)

Gly: 1.08 (1)

*Ala: 3.00 (3)

Pro: 1.07 (1)

Ile: 1.07 (1)

Leu: 1.09 (1)

Trp: 0.94 (1)

*Ala was used as a standard amino acid. The values in parentheses indicate calculated values.

Example 9

Synthesis of [$Pro^4$]HCNP(2-11):

In a solid phase synthesis reactor was charged 0.60 g of HCNP(5-11) peptide resin described in Example 1. Following Schedule 4 described in Example 5, Boc-Pro-OH, Boc-Asp(OcHex)-OH and Boc-Ala-OH were successively coupled with the peptide resin. As the result, 0.61 g of [$Pro^4$]HCNP(2-11) peptide resin was obtained.

To 0.61 g of this [$Pro^4$]HCNP(2-11) peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 15 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. After the filtrate was lyophilized, the resulting crude peptide was dissolved in 50 ml of 10% acetic acid aqueous solution. The solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to about 23% acetonitrile in 180 minutes, to 26% in 60 minutes and further to 28% in 60 minutes, at a flow rate of 7.0 ml/min. The eluent was monitored at A280 nm. The fractions containing the desired product were collected and lyophilized to give 70,6 mg of [$Pro^4$]HCNP(2-11).

The thus obtained [$Pro^4$]HCNP(2-11) was eluted at retention time of 24.4 minutes with linear density gradient of 10-50% aqueous acetonitrile containing 0.1% TFA through reverse phase YMC-AM303(S-5)-ODS column (4.6 ø x 250 mm). The amino acid analysis of the peptide coincided with the calculated values.

Amino acid analysis

| | |
|---|---|
| Hydrolysis: | 4N Methanesulfonic acid, 2% tryptamine, at 110°C for 24 hours |
| Analysis method: | PICO-TAG (reverse phase-PTC amino acid) method |
| Result: | Asx: 0.95 (1) |
| | Glx: 0.98 (1) |
| | Ser: 0.88 (1) |
| | Gly: 0.85 (1) |
| | Ala: 1.95 (2) |
| | Pro: 1.96 (2) |
| | *Leu: 1.00 (1) |
| | Trp: 0.82 (1) |

*Leu was used as a standard amino acid. The values in parentheses indicate calculated values.

Example 10

Synthesis of [Aib$^4$]HCNP(2-11):

In a solid phase synthesis reactor was charged 0.60 g of HCNP(5-11) peptide resin described in Example 1. Following Schedule 4 described in Example 5, Boc-Aib-OH, Boc-Asp(OcHex)-OH and Boc-Ala-OH were successively coupled with the resin. As the result, 0.63 g of [Aib$^4$]HCNP(2-11) peptide resin was obtained.

To 0.63 g of this [Aib$^4$]HCNP(2-11) peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 15 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. After the filtrate was lyophilized, the resulting crude peptide was dissolved in 50 ml of 10% acetic acid aqueous solution. The solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to 24% acetonitrile in 180 minutes, to 27% in 60 minutes and further to 29% in 60 minutes, at a flow rate of 7.0 ml/min. The eluent was monitored at A280 nm. The fractions containing the desired product were collected and lyophilized to give 46.5 mg of [Aib$^4$]HCNP(2-11).

The thus obtained [Aib$^4$]HCNP(2-11) was eluted at retention time of 25.0 minutes with linear density gradient of 10-50% aqueous acetonitrile containing 0.1% TFA through reverse phase YMC-AM303(S-5)-ODS column (4.6 ø x 250 mm). The amino acid analysis of the peptide coincided with the calculated values (but Aib was not detected).

Amino acid analysis

| | |
|---|---|
| Hydrolysis: | 4N Methanesulfonic acid, 2% tryptamine, at 110°C for 24 hours |
| Analysis method: | PICO-TAG (reverse phase-PTC amino acid) method |
| Result: | Asx: 1.05 (1) |
| | Glx: 1.05 (1) |
| | Ser: 0.98 (1) |
| | Gly: 1.04 (1) |
| | *Ala: 2.00 (2) |
| | Pro: 1.05 (1) |
| | Leu: 1.00 (1) |
| | Trp: 0.78 (1) |

*Ala was used as a standard amino acid. The values in parentheses indicate calculated values.

Example 11

Synthesis of [MeIle[4]]HCNP(4-8)$NH_2$:

(1) Synthesis of Boc-Ala-$NH_2$

After 20.0 g of Boc-Ala-OH was dissolved in 300 ml of THF, the solution was cooled to -15°C. Then 10.69 g of N-methylmorpholine and 14.44 g of IBCF were dropwise added to the solution. After stirring for 5 minutes, 31.0 ml of conc. ammonia water was added and the mixture was stirred at -15°C to -10°C for 30 minutes and then at room temperature for further an hour. The precipitated crystals were filtered off. After THF was distilled off, 400 ml of AcOEt was added to the residue and the crystals were further filtered off. The filtrate was washed with saturated sodium hydrogencarbonate aqueous solution and saturated sodium chloride aqueous solution. After the AcOEt layer was desiccated over magnesium sulfate, the solvent was distilled off. Hexane was added to the residue and the formed crystals were filtered and desiccated to give 15.66 g of Boc-Ala-$NH_2$.

(2) Synthesis of Boc-Trp-Ala-$NH_2$

After 6.0 g of Boc-Ala-$NH_2$ was suspended in 53 ml of acetonitrile, a solution of 24.5 g of methanesulfonic acid in 30 ml of acetonitrile was dropwise added to the suspension under ice cooling. After stirring for 1.5 hour, 21 ml of DMF was added to the mixture. The resulting mixture was neutralized with 22.5 g of TEA under ice cooling. To the mixture were added 50 ml of DMF and 50 ml of NMP. Then 5.15 g of HOBt and 10.64 g of Boc-Trp-OH were dissolved in the mixture. Thereafter, 5.92 g of EDC was added to the solution, which was stirred for 30 minutes and then at room temperature for further 2 hours. To the reaction solution was added 400 ml of AcOEt. After washing sequentially with 1N-HCl aqueous solution, saturated sodium hydrogencarbonate aqueous solution and saturated sodium chloride aqueous solution, the reaction solution was desiccated over magnesium sulfate and the solvent was distilled off. After crystallization from hexane, filtration and desication

were performed to give 9.93 g of Boc-Trp-Ala-$NH_2$.

(3) Synthesis of Boc-Gln-Trp-Ala-$NH_2$

After 5.67 g of Boc-Trp-Ala-$NH_2$ was suspended in 60 ml of acetonitrile, 7.39 g of methanesulfonic acid was dropwise added to the suspension under ice cooling. After stirring for 1.5 hour, the resulting mixture was neutralized with 7.78 g of TEA. To the solution were added 4.56 g of Boc-Gln-OH, 2.50 g of HOBt and 50 ml of DMF in sequence to dissolve them. Thereafter, 3.55 g of EDC.HCl was added to the solution. The temperature was elevated to room temperature followed by stirring for an hour. After 30 ml of water was added, the solvent was concentrated in vacuo and 100 ml of water was added to the residue followed by extraction with n-BuOH (50 ml x 3). The n-BuOH layer was collected and washed with 5% sodium hydrogencarbonate aqueous solution (100 ml x 2) and water (50 ml). n-BuOH was distilled off in vacuo. After drying, 9.46 g of Boc-Gln-Trp-Ala-$NH_2$ was obtained.

(4) Synthesis of Boc-Ser(Bzl)-Gln-Trp-Ala-$NH_2$

After 7.00 g of Boc-Gln-Trp-Ala-$NH_2$ was suspended in 100 ml of acetonitrile, 6.69 g of methanesulfonic acid was dropwise added to the suspension under ice cooling. The temperature was elevated to room temperature. After stirring for 3.5 hours, the mixture was neutralized with 7.04 g of TEA. To the solution were added 5.78 g of Boc-Ser(Bzl)-OH, 2.82 g of HOBt and 50 ml of DMF in sequence to dissolve them. Thereafter, 4.00 g of EDC.HCl was added to the solution. The temperature was elevated to room temperature. Stirring was continued for 1.5 hour and 20 ml of water was added to the system. After stirring for 15 minutes, acetonitrile was distilled off and the residue was dropwise added to 1 liter of water. After stirring for 30 minutes, the formed precipitates were filtered, washed with water (200 ml) and desiccated to give 7.35 g of Boc-Ser(Bzl)-Gln-Trp-Ala-$NH_2$.

(5) Synthesis of Boc-MeIle-Ser(Bzl)-Gln-Trp-Ala-$NH_2$

After 0.20 g of Boc-Ser(Bzl)-Gln-Trp-Ala-$NH_2$ was suspended in 5 ml of acetonitrile, 0.20 ml of methanesulfonic acid was added to the suspension under ice cooling. The temperature was elevated to room temperature. After stirring for 2 hours the mixture was neutralized with 0.43 ml of TEA. To the solution were added 87 mg of Boc-MeIle-OH, 48 mg of HOBt and 5 ml of DMF in sequence to dissolve them. Thereafter, 68 mg of EDC.HCl was added to the solution. The temperature was elevated to room temperature. Stirring was continued overnight. Acetonitrile was distilled off and 30 ml of water was added to the residue. After stirring for 30 minutes, the formed precipitates were filtered, washed with water and desiccated to give 0.15 g of Boc-MeIle-Ser(Bzl)-Gln-Trp-Ala-$NH_2$.

(6) Synthesis of TFA.H-MeIle-Ser(Bzl)-Gln-Trp-Ala-$NH_2$

Under ice cooling, 0.14 g of Boc-MeIle-Ser(Bzl)-Gln-Trp-Ala-$NH_2$ was dissolved in TFA-anisole (5 ml + 1 ml). After stirring for an hour, TFA was distilled off in vacuo and 20 ml of diethyl ether was added to the residue to form precipitates. The precipitates were filtered and desiccated to give 0.13 g of TFA.H-MeIle-Ser(Bzl)-Gln-Trp-Ala-$NH_2$.

(7) Synthesis of [$MeIle^4$]HCNP(4-8)$NH_2$

After 0.12 g of TFA.H-MeIle-Ser(Bzl)-Gln-Trp-Ala-$NH_2$ was dissolved in 5 ml of acetic acid-water (2 : 3), 40 mg of 10% Pd/C was added to the solution. The mixture was stirred at room temperature for 7 hours under $H_2$ atmosphere. The reaction solution was diluted with water. The catalyst was filtered off through a membrane filter and the filtrate was lyophilized to give 105 mg of the crude peptide.

The crude peptide was dissolved in 40 ml of 5% acetic acid aqueous solution and the solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to 7% acetonitrile in 180 minutes and then to 10% in 60 minutes and further to 12% in 60 minutes, at a flow rate of 7.0 ml/min. The eluent was monitored at A280 nm. The fractions containing the desired product were collected and lyophilized to give 14.9 mg of [$MeIle^4$]HCNP(4-8)$NH_2$.

The thus obtained [$MeIle^4$]HCNP(4-8)$NH_2$ was eluted at retention time of 15.5 minutes with linear density gradient of 10-50% aqueous acetonitrile containing 0.1% TFA through reverse phase YMC-AM303(S-5)-ODS

column (4.6 ø x 250 mm). The amino acid analysis of the peptide (but Melle was not detected) and mass spectrum coincided with the calculated values.

Amino acid analysis

| | |
|---|---|
| Hydrolysis: | 4N Methanesulfonic acid, 2% tryptamine, at 110°C for 25 hours |
| Analysis method: | PICO-TAG (reverse phase-PTC amino acid) method |
| Result: | Glx: 1.19 (1) |
| | Ser: 0.72 (1) |
| | *Ala: 1.00 (1) |
| | Trp: 0.71 (1) |

*Ala was used as a standard amino acid. The values in parentheses indicate calculated values.

Mass spectrum (SIMS)

$[M + H]^+$: 617

Example 12

Synthesis of [pGlu⁶]HCNP(6-7):

(1) Synthesis of Boc-Gln-Trp-OH

After 17.98 g of Boc-Gln-ONp and 11.99 g of H-Trp-OH were suspended in 260 ml of DMF, 7.43 g of TEA was dropwise added to the suspension under ice cooling. The mixture was stirred overnight. After DMF was distilled off, 10% citric acid aqueous solution was added to the residue followed by extraction with AcOEt 3 times. The AcOEt layer was concentrated in vacuo. The precipitated crystals were filtered, washed with water and AcOEt and then desiccated to give 18.61 g of Boc-Gln-Trp-OH.

(2) Synthesis of TFA.H-Gln-Trp-OH

After 8.0 g of Boc-Gln-Trp-OH and 8 ml of anisole were dissolved in 80 ml of TFA, the solution was stirred at room temperature for 30 minutes. The reaction solution was dropwise added to 800 ml of diethyl ether. The formed precipitates were filtered off. The precipitates were washed with diethyl ether and then desiccated to give 7.86 g of TFA.H-Gln-Trp-OH.

(3) Synthesis of [pGlu⁶]HCNP(6-7)

In 100 ml of water was dissolved 2.5 g of TFA.H-Gln-Trp-OH. The solution was applied to reverse phase YMC-SH-343-5(S-5)ODS column (30 ø x 500 mm) previously equilibrated with 0.1% TFA aqueous solution. After the column was washed with 0.1% TFA aqueous solution, the peptide was eluted with linear gradient of 0 to 11% acetonitrile in 180 minutes and then to 14% in 60 minutes and further to 16% in 60 minutes, at a flow rate of 7.0 ml/min. The eluent was monitored at A280 nm. The fractions containing H-Gln-Trp-OH were collected and acetonitrile was distilled off in vacuo. The residue was allowed to stand in a refrigerator. The precipitated crystals were filtered, washed and desiccated to give 167.23 mg of [pGlu⁶]HCNP(6-7).

The thus obtained [pGlu[6]]HCNP(6-7) was eluted at retention time of 19.5 minutes with linear density gradient of 10-50% aqueous acetonitrile containing 0.1% TFA through reverse phase YMC-AM303(S-5)-ODS column (4.6 ø x 250 mm). The amino acid analysis of the peptide (provided that pGlu was recovered as Glx) and mass spectrum coincided with the calculated values.

Amino acid analysis

Hydrolysis: 4N Methanesulfonic acid, 2% tryptamine, at 110°C for 24 hours

Analysis method: PICO-TAG (reverse phase-PTC amino acid) method

Result: *Glx: 1.00 (1)

Trp: 0.65 (1)

*Glx was used as a standard amino acid. The values in parentheses indicate calculated values.

Mass spectrum (SIMS)

$[M + H]^+$: 316

Example 13

Synthesis of [D-Trp[7]]HCNP:

In a solid phase synthesis reactor was charged 6.0 g of Boc-Leu-O-resin described in Example 1. Following Schedule 3 described in Example 2, Boc-Pro-OH, Boc-Gly-OH and Boc-Ala-OH were successively coupled with the resin. During the course of the coupling 1/4 amount of the resin was taken out at the time when coupling of Boc-Gly-OH was completed, and further 1/3 amount of the resin was taken out after the coupling of Boc-Ala-OH was completed. Thus, 1.69 g of HCNP(8-11) peptide resin was obtained. This HCNP(8-11) peptide resin was further coupled, in sequence, with Boc-D-Trp-OH, Boc-Gln-OH, Boc-Ser(Bzl)-OH, Boc-Ile-OH, Boc-Asp-(OcHex)-OH, Boc-Ala-OH and Boc-Ala-OH, following Schedule 2 described in Example 1. As the result, 2.46 g of [D-Trp[7]]HCNP peptide resin was obtained.

To 1.23 g of this [D-Trp[7]]HCNP peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 30 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. After the filtrate was lyophilized, the resulting crude peptide was dissolved in 200 ml of 2.5% acetic acid aqueous solution. The solution was applied to reverse phase YMC-A363(S-5)ODS column (30 ø x 250 mm) previously equilibrated with 0.1% TFA aqueous solution. After the column was washed with 0.1% TFA aqueous solution, the peptide was eluted with linear gradient of 0 to 14% acetonitrile in 30 minutes, to 24% in 100 minutes and further to 34% in 100 minutes, at a flow rate of 7.0 ml/min. The eluent was monitored at A280 nm. The fractions containing the desired product were collected and lyophilized to give 267.6 mg of primarily purified [D-Trp[7]]HCNP. In 2 ml of water was dissolved 20 mg out of the primarily purified product. The solution was divided into 0.2 ml each and applied in 10 portions to reverse phase YMC-AM323(S-5)ODS column (10 ø x 250 mm). The peptide was eluted with 25% aqueous acetonitrile containing 0.1% TFA. The eluent was monitored at A280 nm. The fractions containing the desired product were collected and lyophilized to give 14.4 mg of [D-Trp[7]]HCNP.

The thus obtained [D-Trp[7]]HCNP was eluted at retention time of 26.7 minutes with linear density gradient of 10-50% aqueous acetonitrile containing 0.1% TFA through reverse phase YMC-AM303(S-5)-ODS column

(4.6 ø x 250 mm). The amino acid analysis of the peptide coincided with the calculated values.

Amino acid analysis

| | |
|---|---|
| Hydrolysis: | 4N Methanesulfonic acid, 2% tryptamine, at 110°C for 24 hours |
| Analysis method: | PICO-TAG (reverse phase-PTC amino acid) method |
| Result: | Asx: 1.07 (1) |
| | Glx: 1.06 (1) |
| | Ser: 0.97 (1) |
| | Gly: 1.09 (1) |
| | *Ala: 3.00 (3) |
| | Pro: 1.09 (1) |
| | Ile: 1.05 (1) |
| | Leu: 1.09 (1) |
| | Trp: 0.85 (1) |

*Ala was used as a standard amino acid. The values in parentheses indicate calculated values.

Example 14

Synthesis of [D-$Trp^1$]HCNP(4-8)$NH_2$:

(1) Synthesis of Boc-D-Trp-Ala-$NH_2$

After 7.53 g of Boc-Ala-$NH_2$ was suspended in 60 ml of acetonitrile, a solution of 23.0 g of methanesulfonic acid in 27 ml of acetonitrile was dropwise added to the suspension under ice cooling. After stirring for 1.5 hour, 30 ml of DMF was added to the mixture. The resulting mixture was neutralized with 20.1 g of TEA under ice cooling. To the mixture were added 50 ml of DMF and 70 ml of NMP. Then 6.44 g of HOBt and 13.31 g of Boc-D-Trp-OH were dissolved in the solution. Thereafter, 7.39 g of EDC was added to the solution, which was stirred for 30 minutes and then at room temperature for further 3 hours. To the reaction solution was added 400 ml of AcOEt. After washing sequentially with 5% citric acid aqueous solution, saturated sodium hydrogencarbonate aqueous solution and saturated sodium chloride aqueous solution, the organic layer was desiccated over magnesium sulfate and the solvent was distilled off. After crystallization from hexane, the crystals were filtered. The crystals were washed with methylene chloride and desiccated to give 10.10 g of Boc-D-Trp-Ala-$NH_2$.

(2) Synthesis of Boc-Gln-D-Trp-Ala-$NH_2$

After 1.0 g of Boc-D-Trp-Ala-$NH_2$ was suspended in 10 ml of acetonitrile, 0.87 ml of methanesulfonic acid was dropwise added to the suspension under ice cooling. The temperature was elevated to room temperature. After stirring for 4.5 hours, the mixture was neutralized with 1.3 ml of TEA. To the solution were added 0.79 g of Boc-Gln-OH, 0.43 g of HOBt and 18 ml of DMF in sequence to dissolve them. Thereafter, 0.61 g of EDC.HCl

was added to the solution. The temperature was elevated to room temperature followed by stirring overnight. After 10 ml of water was added, the solvent was distilled off in vacuo and 20 ml of water was added to the residue followed by extraction with n-BuOH (30 ml x 5). The n-BuOH layer was collected and washed with 5% sodium hydrogencarbonate aqueous solution (50 ml x 5) and water (50 ml). n-BuOH was distilled off in vacuo. After drying, 1.45 g of Boc-Gln-D-Trp-Ala-$NH_2$ was obtained.

(3) Synthesis of Boc-Ser(Bzl)-Gln-D-Trp-Ala-$NH_2$

After 1.0 g of Boc-Gln-D-Trp-Ala-$NH_2$ was suspended in 10 ml of acetonitrile, 0.64 ml of methanesulfonic acid was dropwise added to the suspension under ice cooling. The temperature was elevated to room temperature. After stirring for 3 hours the mixture was neutralized with 1.39 ml of TEA. To the solution were added 0.83 g of Boc-Ser(Bzl)-OH, 0.40 g of HOBt and 15 ml of DMF in sequence to dissolve them. Thereafter, 0.57 g of EDC.HCl was added to the solution. The temperature was elevated to room temperature. Stirring was continued for an hour and 10 ml of water was added to the system. After stirring for 15 minutes, acetonitrile was distilled off and water was added to the residue. The formed precipitates were filtered, washed with water and desiccated to give 1.20 g of Boc-Ser(Bzl)-Gln-D-Trp-Ala-$NH_2$.

(4) Synthesis of Boc-Ile-Ser(Bzl)-Gln-D-Trp-Ala-$NH_2$

After 0.50 g of Boc-Ser(Bzl)-Gln-D-Trp-Ala-$NH_2$ was suspended in 10 ml of acetonitrile, 0.48 ml of methanesulfonic acid was added to the suspension under ice cooling. The temperature was elevated to room temperature. After stirring for 1.5 hour, the mixture was neutralized with 1.20 ml of TEA. To the solution were added 212 mg of Boc-Ile-OH.1/2$H_2O$, 119 mg of HOBt and 15 ml of DMF in sequence to dissolve them. Thereafter, 169 mg of EDC.HCl was further added to the solution. The temperature was elevated to room temperature. Stirring was continued for 2 hours. After 10 ml of water was added to the reaction solution, acetonitrile was distilled off and the residue was dropwise added to 100 ml of water. After stirring for 30 minutes, the formed precipitates were filtered, washed with water and desiccated to give 396 mg of Boc-Ile-Ser(Bzl)-Gln-D-Trp-Ala-$NH_2$.

(5) Synthesis of Boc-Ile-Ser-Gln-D-Trp-Ala-$NH_2$

After 275 mg of Boc-Ile-Ser(Bzl)-Gln-D-Trp-Ala-$NH_2$ was dissolved in 15 ml of acetic acid-DMF (1 : 2), 550 mg of 10% Pd/C was added to the solution. The mixture was stirred at room temperature for 4 hours under $H_2$ atmosphere. The catalyst was filtered off and the solvent was distilled off in vacuo. After drying, 244 mg of Boc-Ile-Ser-Gln-D-Trp-Ala-$NH_2$ was obtained.

(6) Synthesis of [D-$Trp^7$]HCNP(4-8)$NH_2$

Under ice cooling, 237 mg of Boc-Ile-Ser-Gln-D-Trp-Ala-$NH_2$ was dissolved in 5 ml of TFA. After stirring for 50 minutes, TFA was distilled off in vacuo and 150 ml of diethyl ether was added to the residue to form precipitates. The precipitates were filtered and desiccated to give 230 mg of the crude peptide.

The crude peptide was dissolved in 30 ml of water. The solution was applied to reverse phase YMC-SH-343-5(S-5)ODS column (30 ø x 250 mm) previously equilibrated with 0.1% TFA aqueous solution. After the column was washed with 0.1% TFA aqueous solution, the peptide was eluted with linear gradient of 0 to 15% acetonitrile in 180 minutes and then to 18% in 60 minutes and further to 20% in 60 minutes, at a flow rate of 7.0 ml/min. The eluent was monitored at A280 nm. The fractions containing the desired product were collected and lyophilized to give 104.6 mg of [D-$Trp^7$]HCNP(4-8)$NH_2$.

The thus obtained [D-$Trp^7$]HCNP(4-8)$NH_2$ was eluted at retention time of 17.1 minutes with linear density gradient of 10-50% aqueous acetonitrile containing 0.1% TFA through reverse phase YMC-AM303(S-5)-ODS column (4.6 ø x 250 mm). The amino acid analysis of the peptide coincided with the calculated values.

Amino acid analysis

Hydrolysis: 4N Methanesulfonic acid, 2% tryptamine, at 110°C for 25 hours

Analysis method: PICO-TAG (reverse phase-PTC amino acid) method

Result:

| | |
|---|---|
| Glx: | 1.18 (1) |
| Ser: | 1.05 (1) |
| *Ala: | 1.00 (1) |
| Ile: | 1.23 (1) |
| Trp: | 0.33 (1) |

*Ala was used as a standard amino acid. The values in parentheses indicate calculated values.

Example 15

Synthesis of [MeIle$^4$,D-Trp$^7$]HCNP(4-8)NH$(CH_2)_3NH_2$:

(1) Synthesis of Boc-D-Trp(CHO)-Ala-OMe

In 300 ml of DMF were dissolved 10.0 g of HCl.H-Ala-OMe and 23.7 g of Boc-D-Trp(CHO)-OH. Under ice cooling, 10.2 g of HOBt and 11.8 g of EDC were added to the solution. The temperature was elevated to room temperature and the mixture was stirred overnight. Water was added to the reaction solution. After extracting with AcOEt, the extract was washed sequentially with 10% citric acid aqueous solution, saturated sodium hydrogencarbonate aqueous solution and saturated sodium chloride aqueous solution. After drying over magnesium sulfate, the solvent was distilled off. After crystallization from hexane, filtration and desication were performed to give 29.6 g of Boc-D-Trp(CHO)-Ala-OMe.

(2) Synthesis of Boc-Gln-D-Trp(CHO)-Ala-OMe

After 28.6 g of Boc-D-Trp(CHO)-Ala-OMe was suspended in 286 ml of acetonitrile, 23.2 ml of methanesulfonic acid was dropwise added to the suspension under ice cooling. The temperature was elevated to room temperature. After stirring for an hour, the mixture was again ice-cooled and neutralized with 39.7 ml of TEA. To the solution were added 18.5 g of Boc-Gln-OH, 10.1 g of HOBt and 286 ml of DMF in sequence to dissolve them. After 11.6 g of EDC was further added to the solution, the temperature was elevated to room temperature followed by stirring overnight. Acetonitrile was distilled off and the residue was dropwise added to 7 liters of 5% sodium chloride aqueous solution. After stirring for 10 minutes, the mixture was allowed to stand in a dark, cold place. The formed precipitates were filtered and washed sequentially with saturated sodium hydrogencarbonate aqueous solution, water and diethyl ether. After drying, 39.5 g of Boc-Gln-D-Trp(CHO)-Ala-OMe.

(3) Synthesis of Boc-Ser(Bzl)-Gln-D-Trp(CHO)-Ala-OMe

After 25.0 g of Boc-Gln-D-Trp(CHO)-Ala-OMe was suspended in 250 ml of acetonitrile, 14.9 ml of methanesulfonic acid was dropwise added to the suspension under ice cooling. The temperature was elevated to room temperature. After stirring for 3.5 hours, 250 ml of DMF was added to the mixture. The mixture was then neutralized with 25.7 ml of TEA under ice cooling. To the solution were added 14.2 g of Boc-Ser(Bzl)-OH

and 6.50 g of HOBt in sequence to dissolve them. After 7.47 g of EDC was further added to the solution, the temperature was elevated to room temperature followed by stirring for 2.5 hours. Acetonitrile was distilled off and the residue was dropwise added to 4 liters of water. The mixture was allowed to stand in a dark, cold place. The formed precipitates were filtered and washed sequentially with saturated sodium hydrogencarbonate aqueous solution, water and diethyl ether. After drying, 31.6 g of Boc-Ser(Bzl)-Gln-D-Trp(CHO)-Ala-OMe.

(4) Synthesis of Boc-MeIle-Ser(Bzl)-Gln-D-Trp(CHO)-Ala-OMe

After 2.68 g of Boc-Ser(Bzl)-Gln-D-Trp(CHO)-Ala-OMe was suspended in 26 ml of acetonitrile, 1.20 ml of methanesulfonic acid was dropwise added to the suspension under ice cooling. The temperature was elevated to room temperature. After stirring for 2 hours, 20 ml of DMF was added to the mixture. The mixture was then neutralized with 2.09 ml of TEA under ice cooling. To the solution were added 1.0 g of Boc-MeIle-OH, 526 mg of HOBt and 20 ml of DMF in sequence to dissolve them. After 605 mg of EDC was further added to the solution, the temperature was elevated to room temperature followed by stirring overnight. Acetonitrile was distilled off and the residue was dropwise added to 400 ml of water. The formed precipitates were filtered and washed sequentially with saturated sodium hydrogencarbonate aqueous solution, water and diethyl ether. After drying, 2.77 g of Boc-MeIle-Ser(Bzl)-Gln-D-Trp(CHO)-Ala-OMe.

(5) Synthesis of Boc-MeIle-Ser(Bzl)-Gln-D-Trp-Ala-OH

After 2.70 g of Boc-MeIle-Ser(Bzl)-Gln-D-Trp(CHO)-Ala-OMe was dissolved in 54 ml of DMF, 25 ml of IN NaOH was dropwise added to the solution under ice cooling. After stirring for 10 minutes, the reaction solution was poured into 10% citric acid aqueous solution. The formed precipitates were filtered, washed with water and then desiccated to give 2.27 g of Boc-MeIle-Ser(Bzl)-Gln-D-Trp-Ala-OH.

(6) Synthesis of $HCl.H_2N(CH_2)_3NHBoc$

After 5.0 g of $NH_2(CH_2)_3NH_2$ was dissolved in 90 ml of dioxane-water (2 : 1), 15.0 g of TEA was added to the solution. At 15-17°C, 32.39 g of $(Boc)_2O$ was dropwise added to the mixture. After stirring for 7 hours, 200 ml of water was added and the resulting mixture was allowed to stand at a dark cold place. The precipitated crystals were filtered, washed with water and desiccated to give 18.5 g of $BocNH(CH_2)_3NHBoc$. To 50 ml of 1.2N $HCl/Et_2O$ was added 2.0 g of $BocNH(CH_2)_3NHBoc$. The mixture was stirred at room temperature overnight. The formed crystals were filtered, washed with $Et_2O$ and then desiccated to give 1.2 g of $HCl.H_2N(CH_2)_3NHBoc$.

(7) Synthesis of Boc-MeIle-Ser(Bzl)-Gln-D-Trp-Ala-$NH(CH_2)_3NH$-Boc

After 54.8 mg of $HCl.H_2N(CH_2)_3NHBoc$ was suspended in 2 ml of DMF, the suspension was neutralized with 0.04 ml of TEA under ice cooling. To the suspension were added 0.20 g of Boc-MeIle-Ser(Bzl)-Gln-D-Trp-Ala-OH, 35 mg of HOBt and 1 ml of DMF in sequence to dissolve them. Thereafter, 50 mg of EDC.HCl was added to the solution and the temperature was elevated to room temperature followed by stirring overnight. The reaction solution was dropwise added to water. The formed precipitates were filtered and washed with water. After drying, 203 mg of Boc-MeIle-Ser(Bzl)-Gln-D-Trp-Ala-$NH(CH_2)_3NH$-Boc.

(8) Synthesis of H-MeIle-Ser(Bzl)-Gln-D-Trp-Ala-$NH(CH_2)_3NH_2$.2TFA

Under ice cooling, 190 mg of Boc-MeIle-Ser(Bzl)-Gln-D-Trp-Ala-$NH(CH_2)_3NH$-Boc was dissolved in 2.4 ml of TFA and 0.59 ml of anisole. After stirring for an hour, diethyl ether was added to the solution to form precipitates. The precipitates were filtered and desiccated to give 155 mg of H-MeIle-Ser(Bzl)-Gln-D-Trp-Ala-$NH(CH_2)_3NH_2$.2TFA.

(9) Synthesis of H-MeIle-Ser-Gln-D-Trp-Ala-$NH(CH_2)_3NH_2$

After 152 mg of H-MeIle-Ser(Bzl)-Gln-D-Trp-Ala-$NH(CH_2)_3NH_2$.2TFA was dissolved in 8 ml of 50% acetic acid aqueous solution, 75 mg of 10% Pd/C was added to the solution. The mixture was stirred at room temperature for 9.5 hours under $H_2$ atmosphere. The catalyst was filtered off and the filtrate was lyophilized to give 142 mg of H-MeIle-Ser-Gln-D-Trp-Ala-$NH(CH_2)_3NH_2$.

(10) Synthesis of [MeIle⁴,D-Trp⁷]HCNP(4-8)NH$(CH_2)_3NH_2$

In 4 ml of 1 M $NH_4HCO_3$ aqueous solution was dissolved 142 mg of H-MeIle-Ser-Gln-D-Trp-Ala-NH-$(CH_2)_3NH_2$. After stirring at room temperature for 5 hours, pH of the reaction solution was adjusted to 4 with acetic acid, which was then diluted with 96 ml of water. The solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to 6% acetonitrile in 180 minutes and then to 9% in 60 minutes and further to 11% in 60 minutes, at a flow rate of 7.0 ml/min. The eluent was monitored at A280 nm. The fractions containing the desired product were collected and lyophilized to give 15.4 mg of [MeIle$^4$,D-Trp$^7$]HCNP(4-8)NH$(CH_2)_3NH_2$.

The thus obtained [MeIle$^4$,D-Trp$^7$]HCNP(4-8)NH-$(CH_2)_3NH_2$ was eluted at retention time of 15.7 minutes with linear density gradient of 10-50% aqueous acetonitrile containing 0.1% TFA through reverse phase YMC-AM303(S-5)-ODS column (4.6 ø x 250 mm). The amino acid analysis (but MeIle was not detected) of the peptide and mass spectrum coincided with the calculated values.

Amino acid analysis

Hydrolysis: 4N Methanesulfonic acid, 2% tryptamine, at 110°C for 25 hours

Analysis method: PICO-TAG (reverse phase-PTC amino acid) method

Result:
Glx: 1.21 (1)
Ser: 0.66 (1)
*Ala: 1.00 (1)
Trp: 0.83 (1)

*Ala was used as a standard amino acid. The values in parentheses indicate calculated values.

Mass spectrum (SIMS)

$[M + H]^+$: 674

Example 16

Synthesis of [MeIle$^4$,D-Trp$^7$]HCNP(4-8)NH$(CH_2)_3N(CH_3)_2$:

(1) Synthesis of Boc-MeIle-Ser(Bzl)-Gln-D-Trp-Ala-NH$(CH_2)_3N(CH_3)_2$

After 0.03 ml of $H_2N(CH_2)_3N(CH_3)_2$ was dissolved in 3 ml of DMF, 35 mg of HOBt and 50 mg of EDC.HCl were added to the solution. Under ice cooling, 0.20 g of Boc-MeIle-Ser(Bzl)-Gln-D-Trp-Ala-OH described in Example 15 (5) was added to the mixture to dissolve. The solution was stirred overnight. The reaction solution was concentrated in vacuo and water was added to the residue. The formed precipitates were filtered and washed with water. After drying, 376 mg of Boc-MeIle-Ser(Bzl)-Gln-D-Trp-Ala-NH-$(CH_2)_3N(CH_3)_2$ was obtained.

(2) Synthesis of H-MeIle-Ser(Bzl)-Gln-D-Trp-Ala-NH$(CH_2)_3N(CH_3)_2$.2TFA

Under ice cooling, 379 mg of Boc-MeIle-Ser(Bzl)-Gln-D-Trp-Ala-NH$(CH_2)_3N(CH_3)_2$ was dissolved in 2.9 ml of TFA and 0.72 ml of anisole. After stirring for 1.5 hour, diethyl ether was added to the solution to form precipitates. The precipitates were filtered and desiccated to give 310 mg of H-Melle-Ser(Bzl)-Gln-D-Trp-Ala-$NH(CH_2)_3N(CH_3)_2$.2TFA.

(3) Synthesis of [Melle$^4$,D-Trp$^7$]HCNP(4-8)NH-$(CH_2)_3N(CH_3)_2$

After 310 mg of H-Melle-Ser(Bzl)-Gln-D-Trp-Ala-$NH(CH_2)_3N(CH_3)_2$.2TFA was dissolved in 10 ml of 50% acetic acid aqueous solution, 100 mg of 10% Pd/C was added to the solution. The mixture was stirred at room temperature for 8 hours under $H_2$ atmosphere. The catalyst was filtered off and the filtrate was lyophilized. The residue was dissolved in 6.5 ml of 1 M $NH_4HCO_3$ aqueous solution (pH 9). After stirring at room temperature for 5 hours, pH of the reaction solution was adjusted to 4 with acetic acid, which was then diluted with 96 ml of water. The diluted solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to 7% acetonitrile in 180 minutes and then to 10% in 60 minutes and further to 12% in 60 minutes, at a flow rate of 7.0 ml/min. The eluent was monitored at A280 nm. The fractions containing the desired product were collected and lyophilized to give 14.0 mg of [Melle$^4$,D-Trp$^7$]HCNP(4-8)$NH(CH_2)_3N(CH_3)_2$.

The thus obtained [Melle$^4$,D-Trp$^7$]HCNP(4-8)NH-$(CH_2)_3N(CH_3)_2$ was eluted at retention time of 16.1 minutes with linear density gradient of 10-50% aqueous acetonitrile containing 0.1% TFA through reverse phase YMC-AM303(S-5)-ODS column (4.6 ø x 250 mm). The amino acid analysis (but Melle was not detected) of the peptide and mass spectrum coincided with the calculated values.

Amino acid analysis

Hydrolysis: 4N Methanesulfonic acid, 2% tryptamine, at 110°C for 25 hours

Analysis method: PICO-TAG (reverse phase-PTC amino acid) method

Result:
Glx: 1.23 (1)
Ser: 0.70 (1)
*Ala: 1.00 (1)
Trp: 0.74 (1)

*Ala was used as a standard amino acid. The values in parentheses indicate calculated values.

Mass spectrum (SIMS)

$[M + H]^+$: 702

Example 17

Synthesis of [Melle$^4$,D-Trp$^7$]HCNP(4-8)$NH(CH_2)_4$NH-C(=NH)$NH_2$:

(1) Synthesis of $NH_2(CH_2)_4$NHC(=NBoc)NHBoc

After 4 g of methylthioisourea sulfate was dissolved in 80 ml of 5% sodium hydrogencarbonate aqueous solution, 50 ml of methylene chloride was added to the solution. Under ice cooling, 10.1 ml of $(Boc)_2O$ was drop-wise added to the mixture. The temperature was then elevated to room temperature and the mixture was stirred

for 2 days. While stirring, 4 g of sodium hydrogencarbonate, 5 ml of $(Boc)_2O$ and 30 ml of methylene chloride were replenished to the system followed by extraction with methylene chloride. After drying over magnesium sulfate, concentration gave the crude product. The crude product was purified by eluting with hexane-AcOEt (1 : 1) using silica gel column (300 g) to give 1.50 g of $H_3CS$-C(=NBoc)NHBoc. After 0.07 ml of 1,4-diaminobutane was dissolved in 5 ml of THF and 0.1 ml of water, 0.2 g of $H_3CS$-C(=NBoc)NHBoc was added to the solution. The mixture was stirred at 60°C for an hour. AcOEt was added to the reaction mixture. After washing sequentially with saturated sodium hydrogencarbonate aqueous solution and saturated sodium chloride aqueous solution, the AcOEt layer was desiccated over magnesium sulfate and concentrated to give 323 mg of the crude product. The crude product was purified by eluting with AcOEt-MeOH (1 : 2) using silica gel column (25 g) to give 185 mg of $NH_2(CH_2)_4$NHC(=NBoc)NHBoc.

(2) Synthesis of Boc-MeIle-Ser(Bzl)-Gln-D-Trp-Ala-NH$(CH_2)_4$NHC(=NBoc)NHBoc

After 245 mg of $NH_2(CH_2)_4$NHC(=NBoc)NHBoc was dissolved in 4 ml of DMF, 45 mg of HOBt and 65 mg of EDC.HCl were added to the solution. Under ice cooling, 0.20 g of Boc-MeIle-Ser(Bzl)-Gln-D-Trp-Ala-OH described in Example 15 (5) was added to the mixture to dissolve. The solution was stirred for 6.5 hours. The reaction solution was added to water. The formed precipitates were filtered and washed with water. After drying, 281 mg of Boc-MeIle-Ser(Bzl)-Gln-D-Trp-Ala-NH$(CH_2)_4$NHC(=NBoc)NHBoc was obtained.

(3) Synthesis of H-MeIle-Ser(Bzl)-Gln-D-Trp-Ala-NH$(CH_2)_4$NHC(=NH)$NH_2$.TFA

Under ice cooling, 270 mg of Boc-MeIle-Ser(Bzl)-Gln-D-Trp-Ala-NH$(CH_2)_4$NHC(=NBoc)NHBoc was dissolved in 3.5 ml of TFA and 0.80 ml of anisole. After stirring for 2 hours, diethyl ether was added to the solution to form precipitates. The precipitates were filtered and desiccated. Under ice cooling, the precipitates were again dissolved in 3.5 ml of TFA and 0.80 ml of anisole. After stirring for 7 hours, diethyl ether was added to the solution to form precipitates. The precipitates were filtered and desiccated to give 168 mg of H-MeIle-Ser(Bzl)-Gln-D-Trp-Ala-NH$(CH_2)_4$NHC(=NH)$NH_2$.TFA.

(4) Synthesis of [MeIle$^4$,D-Trp$^7$]HCNP(4-8)NH$(CH_2)_4$NH-C(=NH)$NH_2$

After 168 mg of H-MeIle-Ser(Bzl)-Gln-D-Trp-Ala-NH$(CH_2)_4$NHC(=NH)$NH_2$.TFA was dissolved in 10 ml of 50% acetic acid aqueous solution, 80 mg of 10% Pd/C was added to the solution. The mixture was stirred at room temperature for 8 hours under $H_2$ atmosphere. The catalyst was filtered off and the filtrate was lyophilized to give 180 mg of the crude peptide. The crude peptide was dissolved in 30 ml of water. The solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to 8% acetonitrile in 180 minutes and then to 11% in 60 minutes and further to 13% in 60 minutes, at a flow rate of 7.0 ml/min. The eluent was monitored at A280 nm. The fractions containing the desired product were collected and lyophilized to give 22.1 mg of [MeIle$^4$,D-Trp$^7$]HCNP(4-8)NH$(CH_2)_4$NHC(=NH)$NH_2$.

The thus obtained [MeIle$^4$,D-Trp$^7$]HCNP(4-8)-NH$(CH_2)_4$NHC(=NH)$NH_2$ was eluted at retention time of 17.4 minutes with linear density gradient of 10-50% aqueous acetonitrile containing 0.1% TFA through reverse phase YMC-AM303(S-5)-ODS column (4.6 ø x 250 mm). The amino acid analysis (but MeIle was not detected) of the peptide and mass spectrum coincided with the calculated values.

Amino acid analysis

Hydrolysis: 4N Methanesulfonic acid, 2% tryptamine, at 110°C for 25 hours

Analysis method: PICO-TAG (reverse phase-PTC amino acid) method

Result:

Glx: 1.19 (1)

Ser: 0.68 (1)

*Ala: 1.00 (1)

Trp: 0.76 (1)

Agmatine: 0.65 (1)

*Ala was used as a standard amino acid. The values in parentheses indicate calculated values.

Mass spectrum (SIMS)

$[M + H]^+$: 730

Example 18

Synthesis of [$Me_2Ile^4$,D-$Trp^7$]HCNP(4-8)$NH(CH_2)_8NH_2$:

(1) Synthesis of Boc-Ile-Ser(Bzl)-Gln-D-Trp(CHO)-Ala-OMe

After 2.0 g of Boc-Ser(Bzl)-Gln-D-Trp(CHO)-Ala-OMe described in Example 15 (3) was suspended in 20 ml of acetonitrile, 0.90 ml of methanesulfonic acid was dropwise added to the suspension under ice cooling. The temperature was elevated to room temperature. After stirring for 2 hours, 20 ml of DMF was added to the mixture. The mixture was then neutralized with 1.56 ml of TEA under ice cooling. To the solution were added 699 mg of Boc-Ile-OH and 393 mg of HOBt in sequence to dissolve them. After 452 mg of EDC was further added to the solution, the temperature was elevated to room temperature followed by stirring overnight. Acetonitrile was distilled off and the residue was dropwise added to water. The formed precipitates were filtered, washed with water and desiccated to give 2.25 g of Boc-Ile-Ser(Bzl)-Gln-D-Trp(CHO)-Ala-OMe.

(2) Synthesis of H-Ile-Ser(Bzl)-Gln-D-Trp(CHO)-Ala-OMe.TFA

Under ice cooling, 1.0 g of Boc-Ile-Ser(Bzl)-Gln-D-Trp(CHO)-Ala-OMe was dissolved in 20 ml of TFA. After stirring for an hour, TFA was distilled off and diethyl ether was added to the residue to form precipitates. The precipitates were filtered and desiccated to give 1.10 g of H-Ile-Ser(Bzl)-Gln-D-Trp(CHO)-Ala-OMe.TFA.

(3) Synthesis of $Me_2$Ile-Ser(Bzl)-Gln-D-Trp-Ala-OH

After 0.30 g of H-Ile-Ser(Bzl)-Gln-D-Trp(CHO)-Ala-OMe.TFA was dissolved in 6 ml of DMF, 41 mg of $NaBH_3CN$ and then 0.18 ml of 35% HCHO aqueous solution were added to the solution. The temperature was elevated to room temperature and the mixture was stirred for 2.5 hours. During the reaction, 41 mg of $NaBH_3CN$ and 0.35 ml of 35% HCHO aqueous solution were replenished to the system. Further after ice cooling, 3.3 ml of IN NaOH was dropwise added to the solution. The mixture was stirred for 10 minutes and pH was adjusted to 6 with 10% citric acid aqueous solution. The mixture was diluted with water and the diluted solution was

applied to reverse phase YMC-ODS-A120-S30/50 column (40 ø x 60 mm) previously equilibrated with 0.1% TFA aqueous solution. After the column was washed with 0.1% TFA aqueous solution, the peptide was eluted with linear gradient of 0 to 60% acetonitrile. The fractions containing the desired product were collected and lyophilized to give 278 mg of $Me_2$Ile-Ser(Bzl)-Gln-D-Trp-Ala-OH.

(4) Synthesis of HCl.$H_2N(CH_2)_8$NHBoc

After 20.0 g of $NH_2(CH_2)_8NH_2$ was dissolved in 375 ml of dioxane-water (2 : 1), 15.4 g of TEA and 66.5 g of $(Boc)_2O$ were added to the mixture. The temperature was elevated to room temperature followed by stirring overnight. The precipitated crystals were filtered, washed with water and then desiccated to give 37.9 g of BocNH$(CH_2)_8$NHBoc. To 60 ml of 1.2 N HCl/$Et_2O$ was added 2.0 g of BocNH$(CH_2)_8$NHBoc. The mixture was stirred at room temperature for 8 hours. The formed crystals were filtered, washed with $Et_2O$ and desiccated to give 1.27 g of HCl.$H_2N(CH_2)_8$NHBoc.

(5) Synthesis of $Me_2$Ile-Ser(Bzl)-Gln-D-Trp-Ala-NH$(CH_2)_8$NHBoc

After 278 mg of $Me_2$Ile-Ser(Bzl)-Gln-D-Trp-Ala-OH was dissolved in 4.5 ml of DMF, 159 mg of HCl.$H_2N(CH_2)_8$NHBoc, 77 mg of HOBt, 109 mg of EDC.HCl and 0.08 ml of TEA were added to the solution, respectively. The mixture was stirred overnight. While stirring, 0.04 ml of TEA, 25 mg of HOBt and 36 mg of EDC.HCl were replenished to the system. The reaction solution was diluted with 20% acetonitrile aqueous solution. After insoluble matters were filtered off, the filtrate was applied to reverse phase YMC-ODS-Al20-S30/50 column (40 ø x 60 mm) previously equilibrated with 0.1% TFA aqueous solution. After the column was washed with 0.1% TFA aqueous solution, the peptide was eluted with linear gradient of 0 to 70% acetonitrile. The fractions containing the desired product were collected and lyophilized to give 141 mg of $Me_2$Ile-Ser(Bzl)-Gln-D-Trp-Ala-NH$(CH_2)_8$NH-Boc.

(6) Synthesis of [$Me_2$Ile$^4$,D-Trp$^7$]HCNP(4-8)NH$(CH_2)_8NH_2$

To 80 mg of $Me_2$Ile-Ser(Bzl)-Gln-D-Trp-Ala-NH$(CH_2)_8$NH-Boc were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, diethyl ether was added to the residue. The slurry was stirred for 30 minutes, filtered and washed with diethyl ether. The residue was dissolved in water. The solution was lyophilized to give about 80 mg of the crude peptide. The crude peptide was dissolved in 50 ml of water and the solution was applied to reverse phase YMC-SH-363-5(S-5)ODS column (30 ø x 250 mm) previously equilibrated with 0.1% TFA aqueous solution. After the column was washed with 0.1% TFA aqueous solution, the peptide was eluted with linear gradient of 0 to 13% acetonitrile in 180 minutes, to 16% in 60 minutes and further to 18% in 60 minutes, at a flow rate of 7.0 ml/min. The eluent was monitored at A280 nm. The fractions containing the desired product were collected and lyophilized to give 1.67 mg of-primarily purified [$Me_2$Ile$^4$,D-Trp$^7$]HCNP(4-8)NH-$(CH_2)_8NH_2$. In 0.17 ml of water was dissolved 0.38 mg out of the primarily purified product. The solution was applied to reverse phase YMC-AM303(S-5)ODS column (4.6 ø x 250 mm). The peptide was eluted with 23% aqueous acetonitrile containing 0.1% TFA. The eluent was monitored at A220 nm. The fractions containing the desired product were collected and lyophilized to give 0.017 mg of [$Me_2$Ile$^4$,D-Trp$^7$]HCNP(4-8)NH$(CH_2)_8NH_2$.

The thus obtained [$Me_2$Ile$^4$,D-Trp$^7$]HCNP(4-8)NH$(CH_2)_8NH_2$ was eluted at retention time of 22.9 minutes with linear density gradient of 10-50% aqueous acetonitrile containing 0.1% TFA through reverse phase YMC-AM303(S-5)-ODS column (4.6 ø x 250 mm). The structure was confirmed by its amino acid analysis (but $Me_2$Ile was not detected) of the peptide and mass spectrum.

Amino acid analysis

| | |
|---|---|
| Hydrolysis: | 4N Methanesulfonic acid, 2% tryptamine, at 110°C for 24 hours |
| Analysis method: | PICO-TAG (reverse phase-PTC amino acid) method |
| Result: | Glx: 1.16 (1) |
| | Ser: 0.18 (1) |
| | *Ala: 1.00 (1) |
| | Trp: 0.71 (1) |

*Ala was used as a standard amino acid. The values in parentheses indicate calculated values.

Mass spectrum (SIMS)

$[M + H]^+$: 758

Example 19

Synthesis of [$Me_3Ile^4$,D-$Trp^7$]HCNP(4-8)$NH(CH_2)_8NH_2$:

(1) Synthesis of $Me_3$Ile-Ser(Bzl)-Gln-D-Trp-Ala-OH

After 0.50 g of H-Ile-Ser(Bzl)-Gln-D-Trp(CHO)-Ala-OMe.TFA described in Example 18 (2) was dissolved in 10 ml of DMF, $CH_3I$ and TEA were added to the solution by small portions until the reaction was completed, while monitoring the reaction by HPLC. As the result, stirring was continued for 2 days and 5.58 ml of $CH_3I$ and 11.84 ml of TEA were used in total. After ice cooling, 5 ml of 1N NaOH was further added to the mixture followed by stirring for 30 minutes. The reaction mixture was then neutralized with 10% citric acid aqueous solution. After concentration in vacuo, the residue was dissolved in water. The solution was applied to reverse phase YMC-ODS-A120-S10/20 column (28 ø x 260 mm) previously equilibrated with 0.1% TFA aqueous solution. After the column was washed with 0.1% TFA aqueous solution, the peptide was eluted with linear gradient of 0 to 40% acetonitrile. The fractions containing the desired product were collected and lyophilized to give 453 mg of $Me_3$Ile-Ser(Bzl)-Gln-D-Trp-Ala-OH.

(2) Synthesis of $Me_3$Ile-Ser(Bzl)-Gln-D-Trp-Ala-$NH(CH_2)_8$NHBoc

After 200 mg of $Me_3$Ile-Ser(Bzl)-Gln-D-Trp-Ala-OH was dissolved in 3 ml of DMF, 74 mg of $HCl.H_2N(CH_2)_8$NHBoc described in Example 18 (4), 36 mg of HOBt, 51 mg of EDC.HCl and 0.04 ml of TEA were added to the solution, respectively. The mixture was stirred for 8 hours. The reaction solution was concentrated in vacuo and the residue was dissolved in 60 ml of 20% acetonitrile aqueous solution containing 0.1% TFA. The solution was applied to reverse phase YMC-ODS-A120-S10/20 column (28 ø x 260 mm) and the peptide was eluted with linear gradient of 0 to 60% acetonitrile. The fractions containing the desired product were collected and lyophilized to give 220 mg of $Me_3$Ile-Ser(Bzl)-Gln-D-Trp-Ala-$NH(CH_2)_8$NH-Boc.

(3) Synthesis of $Me_3$Ile-Ser(Bzl)-Gln-D-Trp-Ala-$NH(CH_2)_8NH_2$.TFA

Under ice cooling, 200 mg of $Me_3$Ile-Ser(Bzl)-Gln-D-Trp-Ala-$NH(CH_2)_8$NH-Boc was dissolved in 2.5 ml of TFA and 0.63 ml of anisole. After stirring for 30 minutes, diethyl ether was added to the solution to precipitate.

The precipitates were filtered and desiccated to give 160 mg of $Me_3$Ile-Ser(Bzl)-Gln-D-Trp-Ala-NH$(CH_2)_8NH_2$.TFA.

(4) Synthesis of [$Me_3Ile^4$,D-$Trp^7$]HCNP(4-8)NH$(CH_2)_8NH_2$

After 160 mg of $Me_3$Ile-Ser(Bzl)-Gln-D-Trp-Ala-NH$(CH_2)_8NH_2$.TFA was dissolved in 10 ml of 50% acetic acid aqueous solution, 80 mg of 10% Pd/C was added to the solution. The mixture was stirred at room temperature for 8 hours under $H_2$ atmosphere. The catalyst was filtered off and the filtrate was lyophilized to give 200 mg of the crude peptide. The peptide was dissolved in 30 ml of water. The solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to 12% acetonitrile in 180 minutes and then to 15% in 60 minutes and further to 17% in 60 minutes, at a flow rate of 7.0 ml/min. The eluent was monitored at A280 nm. The fractions containing the desired product were collected and lyophilized to give 17.7 mg of [$Me_3Ile^4$,D-$Trp^7$]HCNP(4-8)NH$(CH_2)_8NH_2$.

The thus obtained [$Me_3Ile^4$,D-$Trp^7$]HCNP(4-8)-NH$(CH_2)_8NH_2$ was eluted at retention time of 20.9 minutes with linear density gradient of 10-50% aqueous acetonitrile containing 0.1% TFA through reverse phase YMC-AM303(S-5)-ODS column (4.6 ø x 250 mm). The structure was confirmed by its amino acid analysis (but $Me_3$Ile was not detected) of the peptide and mass spectrum.

Amino acid analysis

Hydrolysis: 4N Methanesulfonic acid, 2% tryptamine, at 110°C for 25 hours

Analysis method: PICO-TAG (reverse phase-PTC amino acid) method

Result:
Glx: 1.18 (1)
Ser: 0.10 (1)
*Ala: 1.00 (1)
Trp: 0.76 (1)

*Ala was used as a standard amino acid. The values in parentheses indicate calculated values.

Mass spectrum (SIMS)

$[M + H]^+$: 773

Example 20

Synthesis of [$AIle^4$,D-$Trp^7$]HCNP(4-8)

(1) Synthesis of $NO_2$-AIle-OH (N-nitroamidino-isoleucine)

After 501 mg of H-Ile-OH was suspended in 8 ml of EtOH-$H_2O$ (1 : 1), 615 mg of N-methyl-N′-nitro-N-nitrosoguanidine was added to the suspension. The mixture was heated to reflux for 8 hours. After allowing to cool, the reaction mixture was diluted with 120 ml of water and neutralized with sodium hydrogencarbonate. The solution was passed through DEAE-Sephadex A-25 column (22 ø x 160 mm). After washing the column with water, the product was eluted with linear gradient of 0 to 30% acetic acid. The fractions containing the product were collected and lyophilized to give 65 mg of $NO_2$-AIle-OH (N-nitro-amidinoisoleucine). Furthermore,

32 mg of $NO_2$-AIle-OH (N-nitroamidinoisoleucine) was obtained in almost the same manner. The yielded amount was 97 mg in total.

(2) Synthesis of $NO_2$-AIle-Ser(Bzl)-Gln-D-Trp(CHO)-Ala-OMe

After 377 mg of Boc-Ser(Bzl)-Gln-D-Trp(CHO)-Ala-OMe described in Example 15 (3) was suspended in 3.8 ml of acetonitrile, 0.17 ml of methanesulfonic acid was dropwise added to the suspension under ice cooling. The temperature was elevated to room temperature. After stirring for an hour, 3.8 ml of DMF was added to the mixture. The reaction mixture was then neutralized with 0.37 ml of TEA under ice cooling. After acetonitrile was distilled off, 95 mg of $NO_2$-AIle-OH (N-nitroamidinoisoleucine) and 64 mg of HOBt were added to the residue in sequence to dissolve. Further after 91 mg of EDC.HCl was added to the solution, pH was adjusted to 9 - 10 with TEA. The temperature was then elevated to room temperature followed by stirring for 2 days. While stirring, 45 mg of EDC.HCl was replenished. After concentrating in vacuo, water was added to the residue. The formed oily substance was taken out and desiccated to give 45 mg of $NO_2$-AIle-Ser(Bzl)-Gln-D-Trp(CHO)-Ala-OMe.

(3) Synthesis of [$AIle^4$,D-$Trp^7$]HCNP(4-8)

To 45 mg of $NO_2$-AIle-Ser(Bzl)-Gln-D-Trp(CHO)-Ala-OMe were added 3 ml of anisole, 0.5 ml of ethylmethyl sulfide and 20 ml of anhydrous hydrogen fluoride. The mixture was reacted at -20°C for 45 minutes and then at 0°C for 60 minutes. After the reaction mixture was concentrated in vacuo, diethyl ether was added to the residue. The slurry was stirred for 30 minutes, filtered and washed with diethyl ether. The residue was dissolved in 20 ml of 15% acetic acid aqueous solution. The solution was lyophilized to give 42 mg of the crude peptide. The crude peptide was dissolved in 5 ml of water and 0.17 ml of 1N NaOH was added to the solution to adjust pH to 10. After stirring for 4 hours, pH was adjusted to 5 with 0.05 ml of acetic acid. The solution was applied by small portions to reverse phase YMC-AM303-(S-5)ODS column (4.6 ø x 250 mm) previously equilibrated with 10% aqueous acetonitrile containing 0.1% TFA. The peptide was eluted with linear gradient of 10 to 40% acetonitrile in 30 minutes, at a flow rate of 1.0 ml/min. The eluent was monitored at A240 nm. The fractions containing the desired product were collected and lyophilized to give 3.6 mg of [$AIle^4$,D-$Trp^7$]HCNP(4-8). The thus obtained [$AIle^4$,D-$Trp^7$]HCNP(4-8) was eluted at retention time of 22.4 minutes with linear density gradient of 10-50% aqueous acetonitrile containing 0.1% TFA through reverse phase YMC-AM303(S-5)-ODS column (4.6 ø x 250 mm). The structure was confirmed by its amino acid analysis (but AIle was not detected) of the peptide and mass spectrum.

Amino acid analysis

| | |
|---|---|
| Hydrolysis: | 4N Methanesulfonic acid, 2% tryptamine, at 110°C for 24 hours |
| Analysis method: | PICO-TAG (reverse phase-PTC amino acid) method |
| Result: | Glx: 1.15 (1) |
| | Ser: 1.05 (1) |
| | *Ala: 1.00 (1) |
| | Trp: 0.75 (1) |

*Ala was used as a standard amino acid. The values in parentheses indicate calculated values.

Mass spectrum (SIMS)

$[M + H]^+$: 646

Example 21

Synthesis of [Cys², Cys⁸]HCNP(1-8)$NH_2$:

In a solid phase synthetic reactor was charged 3.0 g of MBHA resin (crosslinked with 1% divinylbenzene, containing 0.64 mmol of amino group per g of the resin) having a particle diameter of 100 to 200 mesh. Following Schedule 2 described in Example 1, Boc-Cys(4-MeBzl)-OH, Boc-Trp-OH, Boc-Gln-OH and Boc-Ser(Bzl)-OH were successively coupled with the resin. A part of the resin was taken out to obtain about 1 g of [$Cys^8$]HCNP(5-8)$NH_2$ peptide resin. Following Schedule 2, the remaining resin was further coupled with Boc-Ile-OH and a part of the resin was taken out. Following Schedule 2, the remaining resin was further coupled sequentially with Boc-Asp(OcHex)-OH, Boc-Cys(4-MeBzl)-OH and Boc-Ala-OH. As the result, 1.38 g of [$Cys^2$,$Cys^8$]HCNP(1-8)$NH_2$ peptide resin was obtained.

To 1.38 g of this [$Cys^2$,$Cys^8$]HCNP(1-8)$NH_2$ peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 15 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give the crude peptide, which was dissolved in 111 ml of 0.1 M Tris-hydrochloride buffer (pH 8.5) containing 6 M guanidine hydrochloride. The solution was diluted with 590 ml of water followed by stirring for 3 hours. The pH was then adjusted to 5.0 with acetic acid. The solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to 21% acetonitrile in 180 minutes, to 24% in 60 minutes and then to 26% in 60 minutes, at a flow rate of 7.0 ml/min. The eluent was monitored at A280 nm. The fractions containing the desired product were collected and lyophilized to give 18.45 mg of

[$Cys^2$,$Cys^8$]HCNP(1-8)$NH_2$.

The thus obtained

$$[Cys^{2},Cys^{8}]HCNP(1\text{-}8)NH_2$$

was eluted at retention time of 22.2 minutes with linear density gradient of 10-50% aqueous acetonitrile containing 0.1% TFA through reverse phase YMC-AM303(S-5)-ODS column (4.6 ø x 250 mm). The amino acid analysis of the peptide coincided with the calculated values.

Amino acid analysis

Hydrolysis: 4N Methanesulfonic acid, 2% tryptamine, at 110°C for 24 hours

Analysis method: PICO-TAG (reverse phase-PTC amino acid) method

Result:

Asx: 0.96 (1)

Glx: 1.67 (1)

Ser: 1.59 (1)

*Ala: 1.00 (1)

Ile: 0.90 (1)

Trp: 0.70 (1)

Cys: 1.50 (2)

*Ala was used as a standard amino acid. The values in parentheses indicate calculated values.

Mass spectrum (SIMS)

$[M + H]^+$: 922

Example 22

Synthesis of $[Cys^{4},Cys^{8}]HCNP(1\text{-}8)NH_2$:

In a solid phase synthetic reactor was charged about 1 g of $[Cys^{8}]HCNP(5\text{-}8)NH_2$ peptide resin described in Example 21. Following Schedule 4 described in Example 5, Boc-Cys(4-MeBzl)-OH, Boc-Asp(OcHex)-OH, Boc-Ala-OH and Boc-Ala-OH were successively coupled with the resin. As the result, 0.91 g of $[Cys^{4},Cys^{8}]HCNP(1\text{-}8)NH_2$ peptide resin was obtained.

To 0.91 g of this $[Cys^{4},Cys^{8}]HCNP(1\text{-}8)NH_2$ peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added

to the residue. The slurry was stirred for 15 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give the crude peptide, which was dissolved in 111 ml of 0.1 M Tris-hydrochloride buffer (pH 8.5) containing 6 M guanidine hydrochloride. The solution was diluted with 590 ml of water followed by stirring for 2.5 hours. The pH was then adjusted to 5.0 with acetic acid. The solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to 15% acetonitrile in 180 minutes, to 18% in 60 minutes and then to 20% in 60 minutes, at a flow rate of 7.0 ml/min. The eluent was monitored at A280 nm. The fractions containing the desired product were collected and lyophilized to give 14.74 mg of

$$[Cys^4,Cys^8]HCNP(1\text{-}8)NH_2.$$

The thus obtained

$$[Cys^4,Cys^8]HCNP(1\text{-}8)NH_2$$

was eluted at retention time of 15.9 minutes with linear density gradient of 10-50% aqueous acetonitrile containing 0.1% TFA through reverse phase YMC-AM303(S-5)-ODS column (4.6 ø x 250 mm). The amino acid analysis of the peptide and mass spectrum coincided with the calculated values.

Amino acid analysis

Hydrolysis: 4N Methanesulfonic acid, 2% tryptamine, at 110°C for 24 hours

Analysis method: PICO-TAG (reverse phase-PTC amino acid) method

Result:
Asx: 0.98 (1)
Glx: 1.04 (1)
Ser: 0.88 (1)
*Ala: 2.00 (2)
Trp: 0.61 (1)
Cys: 0.96 (2)

*Ala was used as a standard amino acid. The values in parentheses indicate calculated values.

Mass spectrum (SIMS)

$[M + H]^+$: 880

Example 23

Synthesis of Ac[Asp$^3$,Lys$^8$]HCNP(2-8)$NH_2$:

In a solid phase synthetic reactor was charged 4.0 g of MBHA resin (crosslinked with 1% divinylbenzene, containing 0.64 mmol of amino group per g of the resin) having a particle diameter of 100 to 200 mesh. Following Schedule 5 described hereinafter, Boc-Lys(Cl-Z)-OH, Boc-Trp-OH, Boc-Gln-OH, Boc-Ser(Bzl)-OH and Boc-Ile-OH were successively coupled with the resin. About the half of the resin was taken out to obtain about 2 g of [Lys$^8$]HCNP(4-8)$NH_2$ peptide resin. Following Schedule 5, the remaining resin was further coupled sequentially with Boc-Asp(OcHex)-OH and Boc-Ala-OH. About the half of the resin was taken out. The remaining resin was treated by repeating steps 1 to 9 and then steps 16 to 18 to perform deprotection and acetylation. As the result, 1.24 g of Ac[Lys$^8$]HCNP(2-8)$NH_2$ peptide resin was obtained.

To 1.24 g of this Ac[Lys$^8$]HCNP(2-8)$NH_2$ peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 15 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was suspended in 10 ml of DMF. To the suspension were added 1.52 ml of methanesulfonic acid, 3.35 ml of TEA and 140 ml of DMF. The mixture was warmed for 30 minutes to dissolve. After 330 mg of HOBt and 466 mg of EDC.HCl were added to the solution, the mixture was stirred overnight. After concentration in vacuo, the residue was dissolved in 100 ml of 10% acetic acid aqueous solution. The solution was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to 20% acetonitrile in 180 minutes, to 23% in 60 minutes and then to 25% in 60 minutes, at a flow rate of 7.0 ml/min. The eluent was monitored at A280 nm. The fractions containing the desired product were collected and lyophilized to give 3.62 mg of

Ac[Asp$^3$,Lys$^8$]HCNP(2-8)$NH_2$.

The thus obtained

Ac[Asp$^3$,Lys$^8$]HCNP(2-8)$NH_2$

was eluted at retention time of 20.8 minutes with linear density gradient of 10-50% aqueous acetonitrile containing 0.1% TFA through reverse phase YMC-AM303(S-5)-ODS column (4.6 ø x 250 mm). The amino acid analysis of the peptide and mass spectrum coincided with the calculated values.

Amino acid analysis

Hydrolysis: 4N Methanesulfonic acid, 2% tryptamine, at 110°C for 24 hours

Analysis method: PICO-TAG (reverse phase-PTC amino acid) method

Result:
Asx: 0.92 (1)
Glx: 1.09 (1)
Ser: 0.98 (1)
*Ala: 1.00 (1)
Ile: 0.98 (1)
Lys: 0.96 (1)
Trp: 0.38 (1)

*Ala was used as a standard amino acid. The values in parentheses indicate calculated values.

Mass spectrum (SIMS)

$[M + H]^+$: 870

Schedule 5

| Steps | | Time (min.) x Treatment times |
|---|---|---|
| 1. | Washing with methylene chloride, 40 ml | 2 x 3 |
| 2. | Deprotection with 50% TFA, 50% methylene chloride (V/V), 40 ml | 3 x 1<br>20 x 1 |
| 3. | Washing with methylene chloride, 40 ml | 2 x 2 |
| 4. | Washing with methanol, 40 ml | 2 x 2 |
| 5. | Neutralization with 10% triethylamine, 90% methylene chloride (V/V), 40 ml | 1 x 1 |
| 6. | Washing with methanol, 40 ml | 2 x 1 |
| 7. | Neutralization with 10% triethylamine, 90% methylene chloride (V/V), 40 ml | 1 x 1 |
| 8. | Washing with methanol, 40 ml | 2 x 2 |
| 9. | Washing with methylene chloride, 40 ml | 2 x 3 |
| 10. | Coupling by the use of various amino group-protected amino acids (4 mmols), additive (HOBt) 50% DMF-50% methylene chloride (V/V), 20 ml | 5 x 1 |

| | | |
|---|---|---|
| | Solution of DCC (4 mmols) in methylene chloride, 8 ml | 120 x 1 |
| 11. | Washing with 50% DMF, 50% methylene chloride (V/V), 40 ml | 2 x 2 |
| 12. | Washing with methanol, 40 ml | 2 x 1 |
| 13. | Neutralization with 10% triethylamine, 90% methylene chloride (V/V), 40 ml | 1 x 1 |
| 14. | Washing with methanol, 40 ml | 2 x 2 |
| 15. | Washing with methylene chloride, 40 ml | 2 x 2 |
| 16. | Acetylation with 25% acetic anhydride, 75% methylene chloride (V/V), 40 ml | 15 x 1 |
| 17. | Washing with methylene chloride 40 ml | 2 x 2 |
| 18. | Washing with methanol, 40 ml | 2 x 2 |

After the coupling reaction in the step 10, where a small amount of the resin was subjected to ninhydrin test to show positive blue indicative of incompleteness of the coupling reaction, the coupling reaction was repeated using an amino acid of the same protection type.

Example 24

Synthesis of Ac[$Glu^3$,$Lys^8$]HCNP(2-8)$NH_2$:

About 2 g of [$Lys^8$]HCNP(4-8)$NH_2$ peptide resin described in Example 23 was charged in a solid phase synthetic reactor and coupled with Boc-Glu(OcHex)-OH and Boc-Ala-OH following Schedule 5 described in Example 23. About the half of the resin was taken out. The remaining resin was treated by repeating steps 1 to 9 and then steps 16 to 18 to perform deprotection and acetylation. As the result, 0.89 g of Ac[$Glu^3$,$Lys^8$]HCNP(2-8)$NH_2$ peptide resin was obtained.

To 0.89 g of this Ac[$Glu^3$,$Lys^8$]HCNP(2-8)$NH_2$ peptide resin were added 3 ml of anisole, 0.5 ml of ethylmethyl sulfide and 20 ml of anhydrous hydrogen fluoride. The mixture was reacted at -20°C for 60 minutes and then at 0°C for 60 minutes. After the reaction mixture was concentrated in vacuo, 200 ml of diethyl ether was added to the residue. The slurry was stirred for 15 minutes, filtered and washed with 100 ml of diethyl ether. To the residue was added 100 ml of 5% acetic acid aqueous solution. After stirring for 30 minutes, the resin was filtered off and washed with 100 ml of 5% acetic acid aqueous solution. The filtrate was lyophilized to give a crude peptide, which was suspended in 20 ml of DMF. To the suspension were added 1.04 ml of methanesulfonic acid, 2.23 ml of TEA and 30 ml of DMF to dissolve. After 440 mg of HOBt and 620 mg of EDC.HCl were added to the solution, the mixture was stirred for 3.5 hours. After concentration in vacuo, the residue was dissolved in 70 ml of 15% acetic acid aqueous solution and 15 ml of 0.1M Tris-hydrochloride buffer (pH 8.5) containing 6M guanidine hydrochloride.

Insoluble matters were filtered off. After the residue was washed with 30 ml of water, the filtrate was applied to reverse phase YMC-SH-343-5(S-5)ODS column (20 ø x 250 mm) previously equilibrated with 0.1% TFA aqueous solution. After the column was washed with 0.1% TFA aqueous solution, the peptide was eluted with linear gradient of 0 to 19% acetonitrile in 180 minutes, to 22% in 60 minutes and then to 24% in 60 minutes, at a flow rate of 7.0 ml/min. The eluent was monitored at A280 nm. The fractions containing the desired product

were collected and lyophilized to give 27.3 mg of

$$Ac[\overline{Glu^3,Lys^8}]HCNP(2\text{-}8)NH_2.$$

The thus obtained

$$Ac[\overline{Glu^3,Lys^8}]HCNP(2\text{-}8)NH_2$$

was eluted at retention time of 21.4 minutes with linear density gradient of 10-50% aqueous acetonitrile containing 0.1% TFA through reverse phase YMC-AM303(S-5)-ODS column (4.6 ø x 250 mm). The amino acid analysis of the peptide coincided with the calculated values.

Amino acid analysis

Hydrolysis: 4N Methanesulfonic acid, 2% tryptamine, at 110°C for 24 hours

Analysis method: PICO-TAG (reverse phase-PTC amino acid) method

Result:

Glx: 2.14 (2)

Ser: 0.98 (1)

*Ala: 1.00 (1)

Ile: 1.00 (1)

Lys: 1.06 (1)

Trp: 0.45 (1)

*Ala was used as a standard amino acid. The values in parentheses indicate calculated values.

Mass spectrum (FAB)

$[M + H]^+$: 884

Example 25

Assay for biological activity:

The peptides were assayed for a biological activity in accordance with the method of Ojika et al. [Japanese Journal of Psycopharmacology, 7, 447-451 (1987)]. Namely, medial septum nuclei taken out from brain of rat embryo (16 day embryo) is cut to small pieces, then the pieces were cultured in the Bottenstein's modified $N_2$ medium containing 1% FCS in Falcon's 35 mm plastic culture dish with 7% $CO_2$ mixed air, at 36°C. After 3 days culture, the neurotrophic peptide or its derivative was added to the cultures. In a control group, no derivative was added. After 9 days culture, an acetylcholine synthesis activity of cultured tissue was assayed to determine the biological activity of the peptide derivative. The acetylcholine synthesis activity was assayed as follows.

A cultured tissue was pre-incubated in Tyrode solution buffered with Tris-HCl (pH 7.4), then incubated in a buffered solution containing 100 nM [$^3$H] choline chloride (15 Ci/mmol) at 37°C for 30 minutes. After washing out of free [$^3$H] choline, the tissue was dissolved in 1 N formic acid/acetone (15 : 85) solution. Free [$^3$H] choline was converted to phosphocholine by choline kinase (0.1 unit/ml) and [$^3$H] acetylcholine was extracted with tetraphenyl boron (5 mg/ml acetonitrile) to determine the acetylcholine synthesis activity.

EP 91308360.6
Sequence Listing -1-

(1) GENERAL INFORMATION:

(i) APPLICANT:
(A) NAME: Sumitomo Pharmaceutical Company Ltd. et al
(B) STREET: 2-8 Doshomachi-2-chome
(C) CITY: Chuo-ku
(D) STATE: Osaka
(E) COUNTRY: Japan

(ii) TITLE OF INVENTION: Neurotrophic Peptide Derivatives

(iii) NUMBER OF SEQUENCES: 31

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: EP 91308360.6

(2) INFORMATION FOR SEQ ID NO:1:

(i) SEQUENCE CHARACTERISTICS:
(A) LENGTH: 11 amino acids
(B) TYPE: amino acid
(D) TOPOLOGY: linear

(ii) MOLECULE TYPE: peptide

(vi) ORIGINAL SOURCE:
(A) ORGANISM: Homo sapiens

(ix) FEATURE:
(A) NAME/KEY: Peptide
(B) LOCATION: 1..11
(D) OTHER INFORMATION: /label= HCNP
/note= "Hippocampal Cholinergic Neurotrophic Peptide, N terminus optionally modified with a CH3CO- group."

(xi) SEQUENCE DESCRIPTION: SEQ ID NO:1:

```
Ala Ala Asp Ile Ser Gln Trp Ala Gly Pro Leu
1               5                   10
```

EP 91308360.6
Sequence Listing -2-

(2) INFORMATION FOR SEQ ID NO:2:

(i) SEQUENCE CHARACTERISTICS:
(A) LENGTH: 8 amino acids
(B) TYPE: amino acid
(D) TOPOLOGY: linear

(ii) MOLECULE TYPE: peptide

(vi) ORIGINAL SOURCE:
(A) ORGANISM: Homo Sapiens

(ix) FEATURE:
(A) NAME/KEY: Peptide
(B) LOCATION: 1..8
(D) OTHER INFORMATION: /note= "Fragment of HCNP"

(xi) SEQUENCE DESCRIPTION: SEQ ID NO:2:

```
Ala Ala Asp Ile Ser Gln Trp Ala
1               5
```

(2) INFORMATION FOR SEQ ID NO:3:

(i) SEQUENCE CHARACTERISTICS:
(A) LENGTH: 7 amino acids
(B) TYPE: amino acid
(D) TOPOLOGY: linear

(ii) MOLECULE TYPE: peptide

(vi) ORIGINAL SOURCE:
(A) ORGANISM: Homo Sapiens

(ix) FEATURE:
(A) NAME/KEY: Peptide
(B) LOCATION: 1..7
(D) OTHER INFORMATION: /note= "Fragment of HCNP"

(xi) SEQUENCE DESCRIPTION: SEQ ID NO:3:

```
Ala Asp Ile Ser Gln Trp Ala
1               5
```

EP 91308360.6
Sequence Listing -3-

(2) INFORMATION FOR SEQ ID NO:4:

(i) SEQUENCE CHARACTERISTICS:
(A) LENGTH: 7 amino acids
(B) TYPE: amino acid
(D) TOPOLOGY: linear

(ii) MOLECULE TYPE: peptide

(vi) ORIGINAL SOURCE:
(A) ORGANISM: Homo Sapiens

(ix) FEATURE:
(A) NAME/KEY: Peptide
(B) LOCATION: 1..7
(D) OTHER INFORMATION: /note= "Fragment of HCNP"

(xi) SEQUENCE DESCRIPTION: SEQ ID NO:4:

```
Ala Ala Asp Ile Ser Gln Trp
1               5
```

(2) INFORMATION FOR SEQ ID NO:5:

(i) SEQUENCE CHARACTERISTICS:
(A) LENGTH: 10 amino acids
(B) TYPE: amino acid
(D) TOPOLOGY: linear

(ii) MOLECULE TYPE: peptide

(vi) ORIGINAL SOURCE:
(A) ORGANISM: Homo Sapiens

(ix) FEATURE:
(A) NAME/KEY: Peptide
(B) LOCATION: 1..10
(D) OTHER INFORMATION: /note= "Fragment of HCNP"

(xi) SEQUENCE DESCRIPTION: SEQ ID NO:5:

```
Ala Asp Ile Ser Gln Trp Ala Gly Pro Leu
1               5                   10
```

EP 91308360.6
Sequence Listing -4-

(2) INFORMATION FOR SEQ ID NO:6:

(i) SEQUENCE CHARACTERISTICS:
(A) LENGTH: 5 amino acids
(B) TYPE: amino acid
(D) TOPOLOGY: linear

(ii) MOLECULE TYPE: peptide

(vi) ORIGINAL SOURCE:
(A) ORGANISM: Homo Sapiens

(ix) FEATURE:
(A) NAME/KEY: Peptide
(B) LOCATION: 1..5
(D) OTHER INFORMATION: /note= "Fragment of HCNP"

(xi) SEQUENCE DESCRIPTION: SEQ ID NO:6:

```
Ile Ser Gln Trp Ala
1               5
```

(2) INFORMATION FOR SEQ ID NO:7:

(i) SEQUENCE CHARACTERISTICS:
(A) LENGTH: 6 amino acids
(B) TYPE: amino acid
(D) TOPOLOGY: linear

(ii) MOLECULE TYPE: peptide

(vi) ORIGINAL SOURCE:
(A) ORGANISM: Homo Sapiens

(ix) FEATURE:
(A) NAME/KEY: Peptide
(B) LOCATION: 1..6
(D) OTHER INFORMATION: /note= "Fragment of HCNP"

(xi) SEQUENCE DESCRIPTION: SEQ ID NO:7:

```
Ala Asp Ile Ser Gln Trp
1               5
```

EP 91308360.6
Sequence Listing -5-

(2) INFORMATION FOR SEQ ID NO:8:

(i) SEQUENCE CHARACTERISTICS:
(A) LENGTH: 6 amino acids
(B) TYPE: amino acid
(D) TOPOLOGY: linear

(ii) MOLECULE TYPE: peptide

(vi) ORIGINAL SOURCE:
(A) ORGANISM: Homo Sapiens

(ix) FEATURE:
(A) NAME/KEY: Peptide
(B) LOCATION: 1..6
(D) OTHER INFORMATION: /note= "Fragment of HCNP"

(xi) SEQUENCE DESCRIPTION: SEQ ID NO:8:

```
Gln Trp Ala Gly Pro Leu
1               5
```

(2) INFORMATION FOR SEQ ID NO:9:

(i) SEQUENCE CHARACTERISTICS:
(A) LENGTH: 11 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..11
(D) OTHER INFORMATION: /note= "HCNP derivative. Xaa = D-Ala"

(xi) SEQUENCE DESCRIPTION: SEQ ID NO:9:

```
Xaa Ala Asp Ile Ser Gln Trp Ala Gly Pro Leu
1               5                   10
```

EP 91308360.6
Sequence Listing -6-

(2) INFORMATION FOR SEQ ID NO:10:

(i) SEQUENCE CHARACTERISTICS:
(A) LENGTH: 11 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..11
(D) OTHER INFORMATION: /note= "HCNP derivative. Xaa = Ac-Tyr"

(xi) SEQUENCE DESCRIPTION: SEQ ID NO:10:

```
Xaa Ala Asp Ile Ser Gln Trp Ala Gly Pro Leu
1               5                   10
```

(2) INFORMATION FOR SEQ ID NO:11:

(i) SEQUENCE CHARACTERISTICS:
(A) LENGTH: 7 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..7
(D) OTHER INFORMATION: /note= "HCNP derivative; C terminus = $-NH(CH_2)_8NH_2$."

(xi) SEQUENCE DESCRIPTION: SEQ ID NO:11:

```
Arg Ala Asp Ile Ser Gln Trp
1               5
```

EP 91308360.6
Sequence Listing -7-

(2) INFORMATION FOR SEQ ID NO:12:

(i) SEQUENCE CHARACTERISTICS:
(A) LENGTH: 7 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..7
(D) OTHER INFORMATION: /note= "HCNP derivative. Xaa = MeAla; C terminus = $-NH(CH_2)_8NH_2$"

(xi) SEQUENCE DESCRIPTION: SEQ ID NO:12:

Xaa Ala Asp Ile Ser Gln Trp
1 5

(2) INFORMATION FOR SEQ ID NO:13:

(i) SEQUENCE CHARACTERISTICS:
(A) LENGTH: 11 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..11
(D) OTHER INFORMATION: /note= "HCNP derivative.

(xi) SEQUENCE DESCRIPTION: SEQ ID NO:13:

Ala Met Asp Ile Ser Gln Trp Ala Gly Pro Leu
1 5 10

EP 91308360.6
Sequence Listing -8-

(2) INFORMATION FOR SEQ ID NO:14:

(i) SEQUENCE CHARACTERISTICS:
(A) LENGTH: 11 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..11
(D) OTHER INFORMATION: /note= "HCNP derivative. Xaa = Methionine sulphoxide"

(xi) SEQUENCE DESCRIPTION: SEQ ID NO:14:

```
Ala Xaa Asp Ile Ser Gln Trp Ala Gly Pro Leu
1               5                   10
```

(2) INFORMATION FOR SEQ ID NO:15:

(i) SEQUENCE CHARACTERISTICS:
(A) LENGTH: 11 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..11
(D) OTHER INFORMATION: /note= "HCNP derivative. Xaa = Methionine sulphone"

(xi) SEQUENCE DESCRIPTION: SEQ ID NO:15:

```
Ala Xaa Asp Ile Ser Gln Trp Ala Gly Pro Leu
1               5                   10
```

EP 91308360.6
Sequence Listing -9-

(2) INFORMATION FOR SEQ ID NO:16:

(i) SEQUENCE CHARACTERISTICS:
(A) LENGTH: 11 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..11
(D) OTHER INFORMATION: /note= "HCNP derivative.

(xi) SEQUENCE DESCRIPTION: SEQ ID NO:16:

Ala Xaa Gln Ile Ser Gln Trp Ala Gly Pro Leu
1 5 10

(2) INFORMATION FOR SEQ ID NO:17:

(i) SEQUENCE CHARACTERISTICS:
(A) LENGTH: 10 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..10
(D) OTHER INFORMATION: /note= "HCNP derivative.

(xi) SEQUENCE DESCRIPTION: SEQ ID NO:17:

Ala Asp Pro Ser Gln Trp Ala Gly Pro Leu
1 5 10

EP 91308360.6
Sequence Listing -10-

(2) INFORMATION FOR SEQ ID NO:18:

(i) SEQUENCE CHARACTERISTICS:
(A) LENGTH: 10 amino acids
(B) TYPE: amino acid
(D) TOPOLOGY: linear

(ii) MOLECULE TYPE: peptide
(ix) FEATURE:
(A) NAME/KEY: Peptide
(B) LOCATION: 1..10
(D) OTHER INFORMATION: /note= "HCNP derivative. Xaa = 2-Aminoisobutyric acid"

(xi) SEQUENCE DESCRIPTION: SEQ ID NO:18:

```
Ala Asp Xaa Ser Gln Trp Ala Gly Pro Leu
1               5                   10
```

(2) INFORMATION FOR SEQ ID NO:19:

(i) SEQUENCE CHARACTERISTICS:
(A) LENGTH: 5 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..5
(D) OTHER INFORMATION: /note= "HCNP derivative. Xaa = N-Methylisoleucine"

(xi) SEQUENCE DESCRIPTION: SEQ ID NO:19:

```
Xaa Ser Gln Trp Ala
1               5
```

EP 91308360.6
Sequence Listing -11-

(2) INFORMATION FOR SEQ ID NO:20:

(i) SEQUENCE CHARACTERISTICS:
(A) LENGTH: 11 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..11
(D) OTHER INFORMATION: /note= "HCNP derivative. Xaa = D-Trp"

(xi) SEQUENCE DESCRIPTION: SEQ ID NO:20:

```
Ala Ala Asp Ile Ser Gln Xaa Ala Gly Pro Leu
1               5                   10
```

(2) INFORMATION FOR SEQ ID NO:21:

(i) SEQUENCE CHARACTERISTICS:
(A) LENGTH: 5 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..5
(D) OTHER INFORMATION: /note= "HCNP derivative. Xaa = D-Trp"

(xi) SEQUENCE DESCRIPTION: SEQ ID NO:21:

```
Ile Ser Gln Xaa Ala
1               5
```

EP 91308360.6
Sequence Listing -12-

(2) INFORMATION FOR SEQ ID NO:22:

(i) SEQUENCE CHARACTERISTICS:
(A) LENGTH: 5 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..5
(D) OTHER INFORMATION: /note= "HCNP derivative. Xaa(1) = N-Methylisoleucine; Xaa(4) = D-Trp; C terminus = $-NH(CH_2)_3NH_2$."

(xi) SEQUENCE DESCRIPTION: SEQ ID NO:22:

Xaa Ser Gln Xaa Ala
1 5

(2) INFORMATION FOR SEQ ID NO:23:

(i) SEQUENCE CHARACTERISTICS:
(A) LENGTH: 5 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..5
(D) OTHER INFORMATION: /note= "HCNP derivative. Xaa(1) = N-Methylisoleucine; Xaa(4) = D-Trp; C terminus = $-NH(CH_2)_3N(CH_3)_2$."

(xi) SEQUENCE DESCRIPTION: SEQ ID NO:23:

Xaa Ser Gln Xaa Ala
1 5

EP 91308360.6
Sequence Listing -13-

(2) INFORMATION FOR SEQ ID NO:24:

(i) SEQUENCE CHARACTERISTICS:
(A) LENGTH: 5 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..5
(D) OTHER INFORMATION: /note= "HCNP derivative. Xaa(1) = N-Methylisoleucine; Xaa(4) = D-Trp; C terminus = $-NH(CH_2)_4NHC(=NH)NH_2$."

(xi) SEQUENCE DESCRIPTION: SEQ ID NO:24:

Xaa Ser Gln Xaa Ala
1 5

(2) INFORMATION FOR SEQ ID NO:25:

(i) SEQUENCE CHARACTERISTICS:
(A) LENGTH: 5 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..5
(D) OTHER INFORMATION: /note= "HCNP derivative. Xaa(1) = N-Dimethylisoleucine; Xaa(4) = D-Trp; C terminus = $-NH(CH_2)_8NH_2$."

(xi) SEQUENCE DESCRIPTION: SEQ ID NO:25:

Xaa Ser Gln Xaa Ala
1 5

EP 91308360.6
Sequence Listing -14-

(2) INFORMATION FOR SEQ ID NO:26:

(i) SEQUENCE CHARACTERISTICS:
(A) LENGTH: 5 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..5
(D) OTHER INFORMATION: /note= "HCNP derivative. Xaa(1) = N-Trimethylisoleucine; Xaa(4) = D-Trp; C terminus = $-NH(CH_2)_8NH_2$."

(xi) SEQUENCE DESCRIPTION: SEQ ID NO:26:

Xaa Ser Gln Xaa Ala
1 5

(2) INFORMATION FOR SEQ ID NO:27:

(i) SEQUENCE CHARACTERISTICS:
(A) LENGTH: 5 amino acids
(B) TYPE: amino acid
(D) TOPOLOGY: linear

(ii) MOLECULE TYPE: peptide

(ix) FEATURE:
(A) NAME/KEY: Peptide
(B) LOCATION: 1..5
(D) OTHER INFORMATION: /note= "HCNP derivative. Xaa(1) = N-Amidinoisoleucine; Xaa(4) = D-Trp"

(xi) SEQUENCE DESCRIPTION: SEQ ID NO:27:

Xaa Ser Gln Xaa Ala
1 5

EP 91308360.6
Sequence Listing -15-

(2) INFORMATION FOR SEQ ID NO:28:

(i) SEQUENCE CHARACTERISTICS:
(A) LENGTH: 8 amino acids
(B) TYPE: amino acid
(D) TOPOLOGY: cyclic

(ii) MOLECULE TYPE: peptide

(ix) FEATURE:
(A) NAME/KEY: Peptide
(B) LOCATION: 1..8
(D) OTHER INFORMATION: /note= "HCNP derivative. Cys-Cys bond from 2..8"

(xi) SEQUENCE DESCRIPTION: SEQ ID NO:28:

```
Ala Cys Asp Ile Ser Gln Trp Cys
1               5
```

(2) INFORMATION FOR SEQ ID NO:29:

(i) SEQUENCE CHARACTERISTICS:
(A) LENGTH: 8 amino acids
(B) TYPE: amino acid
(D) TOPOLOGY: cyclic

(ii) MOLECULE TYPE: peptide

(ix) FEATURE:
(A) NAME/KEY: Peptide
(B) LOCATION: 1..8
(D) OTHER INFORMATION: /note= "HCNP derivative. Cys-Cys bond from 4..8"

(xi) SEQUENCE DESCRIPTION: SEQ ID NO:29:

```
Ala Ala Asp Cys Ser Gln Trp Cys
1               5
```

EP 91308360.6
Sequence Listing -16-

(2) INFORMATION FOR SEQ ID NO:30:

(i) SEQUENCE CHARACTERISTICS:
(A) LENGTH: 7 amino acids
(B) TYPE: amino acid
(D) TOPOLOGY: cyclic

(ii) MOLECULE TYPE: peptide

(ix) FEATURE:
(A) NAME/KEY: Peptide
(B) LOCATION: 1..7
(D) OTHER INFORMATION: /note= "HCNP derivative. Asp-Lys bond from 2..7; N terminal = Acetyl"

(xi) SEQUENCE DESCRIPTION: SEQ ID NO:30:

```
Ala Asp Ile Ser Gln Trp Lys
1               5
```

(2) INFORMATION FOR SEQ ID NO:31:

(i) SEQUENCE CHARACTERISTICS:
(A) LENGTH: 7 amino acids
(B) TYPE: amino acid
(D) TOPOLOGY: cyclic

(ii) MOLECULE TYPE: peptide

(ix) FEATURE:
(A) NAME/KEY: Peptide
(B) LOCATION: 1..7
(D) OTHER INFORMATION: /note= "HCNP derivative. Glu-Lys bond from 2..7; N terminal = Acetyl"

(xi) SEQUENCE DESCRIPTION: SEQ ID NO:31:

```
Ala Glu Ile Ser Gln Trp Lys
1               5
```

## Claims

1. A neurotrophic peptide derivative represented by the formula (I):

$$A^6 - A^7 \quad (I)$$

[wherein:

(A) in the case where $A^6$ represents $A^5$-Gln and $A^7$ represents Trp-$A^8$;

(a) in the case where:

$A^5$ represents $A^4$-Ser,

$A^4$ represents $A^3$-Ile,

$A^3$ represents $A^2$-Asp and,

$A^2$ represents X-D-Ala-Ala, X-Tyr-Ala, X-Arg-Ala or X-MeAla-Ala;

$A^8$ represents Y or Ala-$A^9$,

(b) in the case where $A^3$ to $A^5$ have the same significances as in (a) and $A^2$ represents $A^1$-Z;

A8 represents Y or Ala-$A^9$,

(c) in the case where $A^3$ to $A^5$ have the same significances as in (a) and $A^2$ represents $A^1$-Cys (provided that $A^1$-Cys forms a cyclic structure together with Cys-$A^9$ of $A^8$ via disulfide bond);

A8 represents Cys-$A^9$ (provided that Cys-$A^9$ forms a cyclic structure together with $A^1$-Cys of $A^2$ via disulfide bond),

(d) in the case where $A^4$ and $A^5$ have the same significances as in (a) and $A^3$ represents $A^2$-Asp (provided that $A^2$-Asp forms a cyclic structure together with Lys-$A^9$ of $A^8$ via amide bond);

$A^2$ represents X, $A^1$-Ala or $A^1$-Z,

$A^8$ represents Lys-A9 (provided that Lys-$A^9$ forms a cyclic structure together with $A^2$-Asp of $A^3$ via amide bond),

(e) in the case where $A^4$ and $A^5$ have the same significances as in (a), $A^3$ represents $A^2$-Gln and $A^2$ represents X, $A^1$-Ala or $A^1$-Z,

$A^8$ represents Y or Ala-$A^9$;

(f) in the case where $A^3$ to $A^5$ have the same significances as in (e) and $A^2$ represents $A^1$-Cys (provided that $A^1$-Cys forms a cyclic structure together with Cys-$A^9$ of $A^8$ via disulfide bond);

$A^8$ represents Cys-$A^9$ (provided that Cys-$A^9$ forms a cyclic structure together with $A^1$-Cys of $A^2$ via disulfide bond),

(g) in the case where $A^4$ and $A^5$ have the same significances as in (a), $A^3$ represents $A^2$-Glu and $A^2$ represents X, $A^1$-Ala or $A^1$-Z

$A^8$ represents Y or Ala-$A^9$,

(h) in the case where $A^3$ to $A^5$ have the same significances as in (g) and $A^2$ represents $A^1$-Cys (provided that $A^1$-Cys forms a cyclic structure together with Cys-$A^9$ of $A^8$ via disulfide bond);

$A^8$ represents Cys-$A^9$ (provided that Cys-$A^9$ forms a cyclic structure together with $A^1$-Cys of $A^2$ via disulphide bond);

(i) in the case where $A^4$ and $A^5$ have the same significances as in (a) and $A^3$ represents $A^2$-Glu (provided that $A^2$-Glu forms a cyclic structure together with Lys-$A^9$ of $A^8$ via amide bond);

$A^2$ represents X, $A^1$-Ala or $A^1$-Z

$A^8$ represents Lye-$A^9$ (provided that Lys-$A^9$ forms a cyclic structure together with $A^2$-Glu of $A^3$ via amide bond),

(j) in the case where $A^5$ has the same significance as in (a),

$A^4$ represents $A^3$-Pro or $A^3$-Aib,

$A^3$ represents X or $A^2$-Gln and

$A^2$ represents X, $A^1$-Ala or $A^1$-Z;

$A^8$ represents Y or Ala-$A^9$,

(k) in the case where $A^3$ to $A^5$ have the same significance as in (j) and $A^2$ represents $A^1$-Cys (provided that $A^1$-Cys forms a cyclic structure together with Cys-$A^9$ of $A^8$ via disulfide bond);

$A^8$ represents Cys-$A^9$ (provided that Cys-$A^9$ forms a cyclic structure together with $A^1$-Cys of $A^2$ via disulfide bond),

(l) in the case where $A^4$ and $A^5$ have the same significances as in (j), $A^3$ represents $A^2$-Asp or $A^2$-Glu and

$A^2$ represents X, $A^1$-Ala or $A^1$-Z,

$A^8$ represents Y or Ala-$A^9$;

(m) in the case where $A^3$ to $A^5$ have the same significances as in (1) and $A^2$ represents $A^1$-Cys (provided that $A^1$-Cys forms a cyclic structure together with Cys-$A^9$ of $A^8$ via disulfide bond);

$A^8$ represents Cys-$A^9$ (provided that Cys-$A^9$ forms a cyclic structure together with $A^1$-Cys of $A^2$ via disulfide bond);

$A^8$ represents Cys-$A^9$ (provided that Cys-$A^9$ forms a cyclic structure together with $A^1$-Cys of $A^2$ via disulfide bond),

(n) in the case where $A^4$ and $A^5$ have the same significance as in (j) and $A^3$ represents $A^2$-Asp (provided that $A^2$-Asp forms a cyclic structure together with Lys-$A^9$ of $A^8$ via amide bond) or $A^2$-Glu (pro-

vided that $A^2$-Glu forms a cyclic structure together with Lys-$A^9$ of $A^8$ via amide bond);
$A^2$ represents X, $A^1$-Ala or $A^1$-Z,
$A^8$ represents Lys-$A^9$ (provided that Lys-$A^9$ forms a cyclic structure together with $A^2$-Asp or $A^2$-Glu of $A^3$ via amide bond),
(o) in the case where $A^5$ has the same significance as in (a) and $A^4$ represents X-MeIle, $Me_2$Ile, $Me_3$Ile or AIle (N-amidinoisoleucine);
$A^8$ represents Y or Ala-$A^9$,
(p) in the case where $A^5$ has the same significance as in (a) and $A^4$ represents $A^3$-Cys (provided that $A^3$-Cys forms a cyclic structure together with Cys-$A^9$ of $A^8$ via disulfide bond);
$A^3$ represents X, $A^2$-Asp, $A^2$-Gln or $A^2$-Glu,
$A^2$ represents X, $A^1$-Ala or $A^1$-Z,
$A^8$ represents Cys-$A^9$ (provided that Cys-$A^9$ forms a cyclic structure together with $A^3$ of $A^4$ via a disulfide bond),

(B) in the case where $A^6$ represents $A^5$-Gln and $A^7$ represents D-Trp-$A^8$;
(a) in the case where:
$A^5$ represents X or $A^4$-Ser,
$A^4$ represents $A^3$-Ile, $A^3$-Pro or $A^3$-Aib,
$A^3$ represents X or $A^2$-Gln and
$A^2$ represents X, $A^1$-Ala or $A^1$-Z;
$A^8$ represents Y of Ala-$A^9$,
(b) in the case where $A^3$ to $A^5$ have the same significance as in (a) and $A^2$ represents $A^1$-Cys (provided that $A^1$-Cys forms a cyclic structure together with Cys-$A^9$ of $A^8$ via disulfide bond);
$A^8$ represents Cys-$A^9$ (provided that Cys-$A^9$ forms a cyclic structure together with $A^1$-Cys of $A^2$ via disulfide bond)
(c) in the case where $A^4$ and $A^5$ have the same significance as in (a), $A^3$ represents $A^2$-Asp or $A^2$-Glu and
A2 represents X, $A^1$-Ala or $A^1$-Z,
$A^8$ represents Y or Ala-$A^9$,
(d) in the case where $A^3$ to $A^5$ have the same significances as in (c) and $A^2$ represents $A^1$-Cys (provided that $A^1$-Cys forms a cyclic structure together with Cys-$A^9$ of $A^8$ via disulfide bond);
$A^8$ represents Cys-$A^9$ (provided that Cys-$A^9$ forms a cyclic structure together with $A^1$-Cys of $A^2$ via disulfide bond),
(e) in the case where $A^4$ and $A^5$ have the same significances as in (a) and $A^3$ represents $A^2$-Asp (provided that $A^2$-Asp forms a cyclic structure together with Lys-$A^9$ of $A^8$ via amide bond) or $A^2$-Glu (provided that $A^2$-Glu forms a cyclic structure together with Lys-$A^9$ of $A^8$ via amide bond);
$A^2$ represents X, $A^1$-Ala or $A^1$-Z,
$A^8$ represents Lys-$A^9$ (provided that Lys-$A^9$ forms a cyclic structure together with $A^2$-Asp or $A^2$-Glu of $A^3$ via amide bond),
(f) in the case where $A^5$ has the same significance as in (a) and
$A^4$ represents X, X-MeIle, $Me_2$Ile, $Me_3$Ile or AIle (N-amidinoisoleucine);
$A^8$ represents Y or Ala-$A^9$,
(g) in the case where
$A^5$ has the same significance as in (a) and $A^4$ represents $A^3$-Cys (provided that $A^3$-Cys forms a cyclic structure together with Cys-$A^9$ of $A^8$ via disulfide bond);
$A^3$ represents X, $A^2$-Asp, $A^2$-Gln or $A^2$-Glu,
$A^2$ represents X, $A^1$-Ala or $A^1$-Z,
$A^8$ represents Cys-$A^9$ (provided that Cys-$A^9$ forms a cyclic structure together with $A^3$-Cys of $A^4$ via disulfide bond),

(C) in the case where $A^6$ represents pGlu and $A^7$ represents Trp-$A^8$ or D-Trp-$A^8$;
$A^8$ represents Y or Ala-$A^9$,

and in all cases of (A), (B) and (C)
$A^7$ is selected from X, X-Ala, X-D-Ala, X-Tyr, X-Arg or X-MeAla
$A^9$ is selected from Y, Gly-Y, Gly-Pro-Y and Gly-Pro-Leu-Y;
X is hydrogen, acyl, sulfonyl or a residue for forming a urea skeleton or urethane skeleton together with an amino group of the amino acid residue to which X is bound;
Y is hydroxyl or a residue for forming an amide group or an ester group together with a carbonyl group of the amino acid residue to which Y is bound and
Z is Met, Met(O) or Met($O_2$);

or a pharmaceutically acceptable salt thereof.

2. A neurotrophic peptide derivative or pharmaceutically acceptable salt thereof according to claim 1, wherein $A^7$ is D-Trp-$A^8$.

3. A neurotrophic peptide derivative or pharmaceutically acceptable salt thereof according to claim 2, wherein $A^3$ is X or $A^4$ is X-MeIle, $Me_2$Ile, $Me_3$Ile or AIle (N-amidinoisoleucine), and $A^9$ is Y.

4. A neurotrophic peptide derivative or pharmaceutically acceptable salt thereof according to claim 3, wherein Y is $NH_2$, $NH(CH_2)_nNH_2$, $NH(CH_2)_n(CH_3)_2$ or $NH(CH_2)_nNHC(=NH)NH_2$ (wherein n represents an integer of 2 to 8).

5. A neurotrophic peptide derivative or pharmaceutically acceptable salt thereof according to claim 1, wherein $A^5$ is X or $A^6$ is pGlu, and $A^8$ is Y.

6. A neurotrophic peptide derivative or pharmaceutically acceptable salt thereof according to claim 1 or claim 2 wherein $A^8$ is Cys-$A^9$ (provided that Cys-$A^9$ forms a cyclic structure together with $A^1$-Cys of $A^2$ or $A^3$-Cys of $A^4$ via disulfide bond); and $A^2$ is $A^1$-Cys (provided that $A^1$-Cys forms a cyclic structure together with Cys-$A^9$ of $A^8$ via disulfide bond); or $A^4$ is $A^3$-Cys (provided that $A^3$-Cys forms a cyclic structure together with Cys-$A^9$ of $A^8$ via disulfide bond).

7. A neurotrophic peptide derivative or pharmaceutically acceptable salt thereof according to claim 1 or claim 2 wherein $A^8$ is Lys-$A^9$ (provided that Lys-$A^9$ forms a cyclic structure together with $A^2$-Asp or $A^2$-Glu of $A^3$ via amide bond) and $A^3$ is $A^2$-Asp (provided that $A^2$-Asp forms a cyclic structure together with Lys-$A^9$ of $A^8$ via amide bond), or $A^2$-Glu (provided that $A^2$-Glu forms a cyclic structure together with Lys-$A^9$ of $A^8$ via amide bond).

8. H-Ala-Ala-Asp-Ile-Ser-Gln-D-Trp-Ala-Gly-Pro-Leu-OH or pharmaceutically acceptable salt thereof.

9. H-Ile-Ser-Gln-D-Trp-Ala-$NH_2$ or pharmaceutically acceptable salt thereof.

10. H-MeIle-Ser-Gln-D-Trp-Ala-$NH(CH_2)_3NH_2$ or pharmaceutically acceptable salt thereof.

11. H-MeIle-Ser-Gln-D-Trp-Ala-$NH(CH_2)_3N(CH_3)_2$ or pharmaceutically acceptable salt thereof.

12. H-MeIle-Ser-Gln-D-Trp-Ala-$NH(CH_2)_4NHC(=N H)NH_2$ or pharmaceutically acceptable salt thereof.

13. $Me_2$Ile-Ser-Gln-D-Trp-Ala-$NH(CH_2)_8NH_2$ or pharmaceutically acceptable salt thereof.

14. $Me_3$Ile-Ser-Gln-D-Trp-Ala-$NH(CH_2)_8NH_2$ or pharmaceutically acceptable salt thereof.

15. pGlu-Trp-OH or pharmaceutically acceptable salt thereof.

16.

H-Ala-Cys-Asp-Ile-Ser-Gln-Trp-Cys-$NH_2$

or pharmaceutically acceptable salt thereof.

17.

H-Ala-Ala-Asp-Cys-Ser-Gln-Trp-Cys-$NH_2$

or pharmaceutically acceptable salt thereof.

**18.**

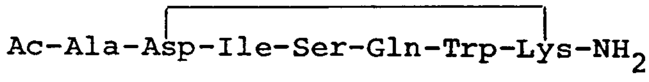

or pharmaceutically acceptable salt thereof.

**19.**

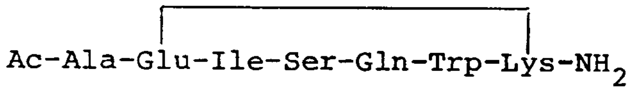

or pharmaceutically acceptable salt thereof.

**20.** A pharmaceutical composition for the treatment of neuro-degenerative disorders comprising as an active ingredient a neurotrophic peptide derivative or pharmaceutically acceptable salt thereof according to any one of claims 1 to 19.

**21.** A pharmaceutical composition for the treatment of dementia comprising as an active ingredient a neurotrophic peptide derivative or pharmaceutically acceptable salt thereof according to any one of claims 1 to 19.

**22.** A pharmaceutical composition for enhancing acetylcholine synthesis in cholinergic neuron in a mammal, which comprises as an active ingredient a neurotrophic peptide derivative or pharmaceutically acceptable salt thereof according to any one of claims 1 to 19.

**23.** A method for preparing the neurotrophic peptide derivative or pharmaceutically acceptable salt thereof according to any one of claims 1 to 19, which comprises chemical synthesis.

**24.** A method according to claim 23, wherein the chemical synthesis is a conventional solid or liquid phase method.

**25.** A method for enhancing acetylcholine synthesis in cholinergic neuron in a mammal, which comprises administering to the mammal an effective amount of a neurotrophic peptide derivative or pharmaceutically acceptable salt thereof according to any one of claims 1 to 19.

**26.** A neurotrophic peptide derivative or pharmaceutically acceptable salt thereof according to any one of claims 1 to 19 for use in a method of treatment of, or of diagnosis practiced on, the human or animal body.

**27.** Use of a neurotrophic peptide derivative or pharmaceutically acceptable salt thereof according to any one of claims 1 to 19 in the preparation of a medicament for use in a method of treatment of, or of diagnosis practiced on, the human or animal body.

European Patent Office

# PARTIAL EUROPEAN SEARCH REPORT

which under Rule 45 of the European Patent Convention shall be considered, for the purposes of subsequent proceedings, as the European search report

Application number: EP 91308360.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,P, X | EP - A - 0 390 602 (SUMITOMO PHARMACEUTICALS COMPANY) * Claims 1,11-36 * & JP-A-3-72 499 ---- | 1, 8-24, 26,27 | C 07 K 7/06<br>A 61 K 37/02 |

TECHNICAL FIELDS SEARCHED (Int. Cl.5)

C 07 K
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-24,26,27
Claims searched incompletely: –
Claims not searched: 25
Reason for the limitation of the search:

Article 52(4) EPC

| Place of search | Date of completion of the search | Examiner |
|---|---|---|
| VIENNA | 12-12-1991 | WOLF |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03.82